# EUROPEAN PATENT SPECIFICATION

(11) **EP 1 179 084 B1**
(45) Date of publication and mention of the grant of the patent: **03.10.2007**
(21) Application number: 00945875.3
(22) Date of filing: 05.07.2000
(51) Int. Cl.: C12P 13/08, C12P 13/06, C12N 15/77

(54) **PROCESS FOR THE FERMENTATIVE PREPARATION OF L-AMINO ACIDS WITH AMPLIFICATION OF THE TKT GENE**
VERFAHREN ZUR FERMENTATIVEN HERSTELLUNG VON L-AMINOSÄUREN DURCH VERSTÄRKUNG DES TKT-GENS
OBTENTION PAR FERMENTATION D'ACIDES L-AMINES AVEC AMPLIFICATION DU GENE TKT

(30) Priority: 17.03.2000 US 528196
(43) Date of publication of application: 13.02.2002
(73) Proprietor: Degussa GmbH, 40474 Düsseldorf (DE); National University of Ireland, Galway (IE)
(72) Inventor: DUNICAN, L., K., (IE); MCCORMACK, Ashling, Athlone, County Westmeath (IE); STAPELTON, Cliona, Roscrea, County Tipparary (IE); BURKE, Kevin, Newcastle, Galway, County Galway (IE); MÖCKEL, Bettina, D-40597 Düsseldorf (DE); THIERBACH, Georg, D-33613 Bielefeld (DE)
(86) International application number: PCT/EP2000/006305
(87) International publication number: WO 2001/068894

(56) References cited:
- US-A- 5 605 818
- US-A- 5 776 736
- M. IKEDA ET AL.: "Cloning of the transketolase gene and the effect of its dosage on aromatic amino acid production in Corynebacterium glutamicum" APPLIED MICROBIOLOGY AND BIOTECHNOLOGY, vol. 51, 1999, pages 201-206, XP000960910 cited in the application
- MASATO IKEDA ET AL.: "Hyperproduction of Tryptophan by Corynebacterium glutamicum with the modified pentose phosphate pathway" APPLIED AND ENVIRONMENTAL MICROBIOLOGY, vol. 65, no. 6, June 1999 (1999-06), pages 2497-2502, XP002153369
- BERNHARD J. EIKMANNS ET AL.: "A family of Corynebacterium glutamicum/Escherichia coli shuttle vectors for cloning, controlled gene expression, and promoter probing" GENE, vol. 102, 1991, pages 93-98, XP002153371 AMSTERDAM NL cited in the application
- BERNHARD J. EIKMANNS: "Identification, sequence analysis, and expression of a Corynebacterium glutamicum gene cluster encoding the three glycolytic enzymes Glyceraldehyde-3-Phosphate dehydrogenase, 3-Phosphoglycerate kinase, and Triosephosphate isomerase" JOURNAL OF BACTERIOLOGY, vol. 174, no. 19, October 1992 (1992-10), pages 6076-6086, XP000979491

## Description

The invention relates to a process for the fermentative preparation of L-lysine using coryneform bacteria in which at least the tkt gene is amplified.

### Prior art

L-Lysine, L-threonine and L-isoleucine are used in animal nutrition, in human medicine and in the pharmaceuticals industry.

It is known that these amino acids are prepared by fermentation of strains of coryneform bacteria, in particular Corynebacterium glutamicum. Because of the great importance, work is constantly being undertaken to improve the preparation processes. Improvements to the processes can relate to fermentation measures, such as e. g. stirring and supply of oxygen, or the composition of the nutrient media, such as e. g. the sugar concentration during the fermentation, or the working up to the product form by e. g. ion exchange chromatography, or the intrinsic output properties of the microorganism itself.

Methods of mutagenesis, selection and mutant selection are used to improve the output properties of these microorganisms. Strains which are resistant to antimetabolites, such as e. g. the threonine analogue **α-**amino-β-hydroxyvaleric acid (AHV), or are auxotrophic for metabolites of regulatory importance and produce L-amino acids such as e. g. threonine are obtained in this manner.

Methods of the recombinant DNA technique have also been employed for some years for improving the strain of Corynebacterium glutamicum strains which produce L-amino acid.

### Object of the invention

The inventors had the object of providing new fundamentals for improved processes for the fermentative preparation of L-lysinewith coryneform bacteria.

### Description of the invention

L-Lysine is used in human medicine and in the pharmaceuticals industry, in the foodstuffs industry and especially in animal nutrition. There is therefore a general interest in providing new improved processes for the preparation of L-Lysine.

The invention provides a process for the fermentative preparation of L-lysine using coryneform bacteria in which the nucleotide sequence which codes for the enzyme transketolase (EC number 2.2.1.1) (tkt gene) is amplified, in particular over-expressed.

The strains employed preferably already produce L-lysine before amplification of the tkt gene.

Preferred embodiments are to be found in the claims.

The term "amplification" in this connection describes the increase in the intracellular activity of one or more enzymes in a microorganism which are coded by the corresponding DNA, for example by increasing the number of copies of the gene or genes, using a potent promoter or using a gene which codes for a corresponding enzyme having a high activity, and optionally combining these measures.

The microorganisms which the present invention provides can prepare L-lysine from glucose, sucrose, lactose, fructose, maltose, molasses, starch, cellulose or from glycerol and ethanol. They are representatives of coryneform bacteria, in particular of the genus Corynebacterium. Of the genus Corynebacterium, there may be mentioned in particular the species Corynebacterium glutamicum, which is known among specialists for its ability to produce L-amino acids.

Suitable strains of the genus Corynebacterium, in particular of the species Corynebacterium glutamicum, are, for example, the known wild-type strains
Corynebacterium glutamicum ATCC13032
Corynebacterium acetoglutamicum ATCC15806
Corynebacterium acetoacidophilum ATCC13870
Corynebacterium thermoaminogenes FERM BP-1539
Brevibacterium flavum ATCC14067
Brevibacterium lactofermentum ATCC13869
Brevibacterium divaricatum ATCC14020
and L-lysine -producing mutants prepared therefrom,
such as, for example, the L-lysine-producing strains
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464
Corynebacterium glutamicum ATCC13032
Corynebacterium glutamicum DM58-1
Corynebacterium glutamicum DSM12866.

It has been found that coryneform bacteria produce L-lysine in an improved manner after over-expression of the tkt gene, which codes for transketolase (EC number 2.2.1.1).

The nucleotide sequence of the tkt gene is disclosed under accession number AB023377 in the databank of the European Molecular Biology Laboratories (EMBL, Heidelberg, Germany). Ikeda et al. (Applied Microbiology and Biotechnology 51, 201-206 (1999) furthermore describe the effect of amplification of the tkt gene on the formation of L-tryptophan, L-tyrosine and L-phenylalanine. As a result of the amplification of the tkt gene in a tryptophan and lysine coproducer the tryptophan production is increased and the lysine production is decreased.

The tkt gene described in the text references mentioned can be used according to the invention. Alleles of the tkt gene which result from the degeneracy of the genetic code or due to sense mutations of neutral function can furthermore be used.

To achieve an amplification (e. g. over-expression), e. g. the number of copies of the corresponding genes is increased, or the promoter and regulation region or the ribosome binding site upstream of the structural gene is mutated. Expression cassettes which are incorporated upstream of the structural gene act in the same way. By inducible promoters, it is additionally possible to increase the expression in the course of fermentative L-lysine formation. The expression is likewise improved by measures to prolong the life of the m-RNA. Furthermore, the enzyme activity is also increased by preventing the degradation of the enzyme protein. The genes or gene constructs are either present here in plasmids with a varying number of copies, or are integrated and amplified in the chromosome. Alternatively, an over-expression of the genes in question can furthermore be achieved by changing the composition of the media and the culture procedure.

Instructions in this context can be found by the expert, inter alia, in Martin et al. (Bio/Technology 5, 137-146 (1987)), in Guerrero et al. (Gene 138, 35-41 (1994)), Tsuchiya and Morinaga (Bio/Technology 6, 428-430 (1988)), in Eikmanns et al. (Gene 102, 93-98 (1991)), in European Patent Specification EPS 0 472 869, in US Patent 4,601,893, in Schwarzer and Pühler (Bio/Technology 9, 84-87 (1991), in Reinscheid et al. (Applied and Environmental Microbiology 60, 126-132 (1994)), in LaBarre et al. (Journal of Bacteriology 175, 1001-1007 (1993)), in Patent Application WO 96/15246, in Malumbres et al. (Gene 134, 15-24 (1993)), in Japanese Laid-Open Specification JP-A-10-229891, in Jensen and Hammer (Biotechnology and Bioengineering 58, 191-195 (1998)) and in known textbooks of genetics and molecular biology.

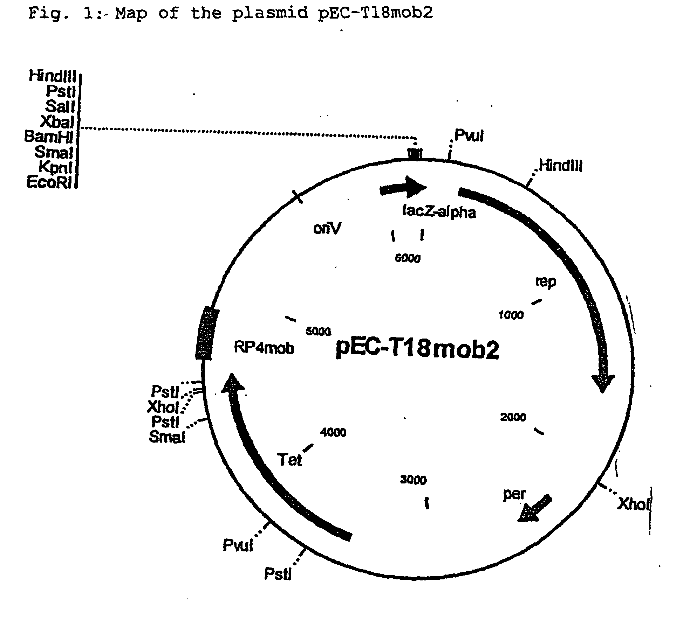

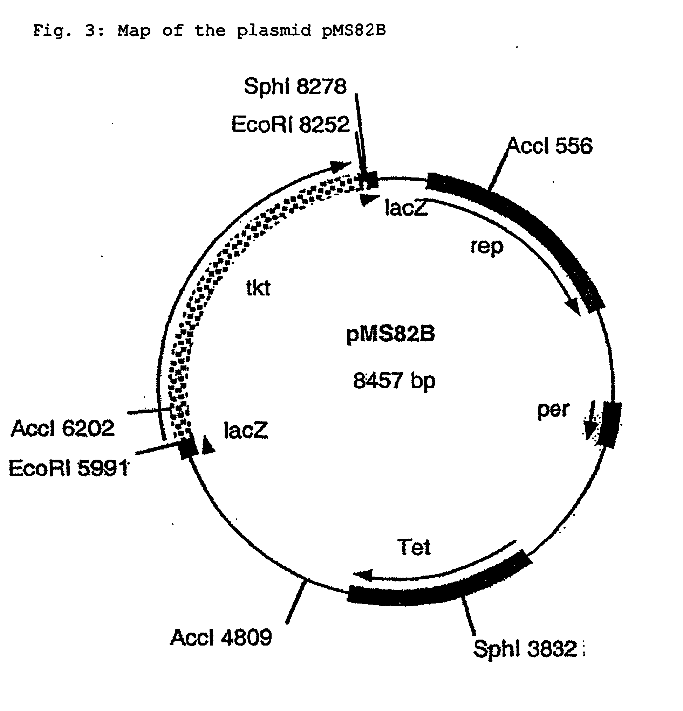

By way of example, transketolase was over-expressed with the aid of a plasmid. The E. coli - C. glutamicum shuttle vector pEC-T18mob2 shown in Figure 1 was used for this. After incorporation of the tkt gene into pEC-T18mob2 and subsequent orientation correction of the DNA fragment carrying the tkt gene, the plasmid pMS82B shown in Figure 3 was formed.

Other plasmid vectors which are capable of replication in C. glutamicum, such as e.g. pEKEx1 (Eikmanns et al., Gene 102:93-98 (1991)) or pZ8-1 (EP-B- 0 375 889), can be used in the same way.

In addition, it may be advantageous for the production of L-lysine to amplify one or more enzymes of the particular biosynthesis pathway, of glycolysis, of anaplerosis or of L-lysine export, in addition to amplification of the tkt gene, which codes for transketolase.

Thus, for the preparation of L-lysine, one or more genes chosen from the group consisting of:
- the dapA gene which codes for dihydrodipicolinate synthase (EP-B 0 197 335),
- the zwf gene which codes for glucose 6-phosphate dehydrogenase (JP-A-09224661),
- at the same time the gnd gene which codes for 6-phosphogluconate dehydrogenase (JP-A-9-224662),
- the eno gene which codes for enolase (DE-A-199 47 791) can be amplified, preferably over-expressed, at the same time.

It may furthermore be advantageous for the production of at the same time to attenuate one or more of the genes chosen from the group consisting of:
- the pck gene which codes for phosphoenol pyruvate carboxykinase (DE-A-199 50 409; DSM 13047),
- the pgi gene which codes for glucose 6-phosphate isomerase (EP-A-1 087 015, DSM-12969),
- the poxB gene which codes for pyruvate oxidase (DE-A-199 51 975; DSM 13114),
in addition to the amplification of the tkt gene.

In addition to over-expression of transketolase, it may furthermore be advantageous for the production of L-lysine to eliminate undesirable side reactions (Nakayama: "Breeding of Amino Acid-Producing Micro-organisms", in: Overproduction of Microbial Products, Krumphahzl, Sikyta, Vanek (eds.), Academic Press, London, UK, 1982).

The microorganisms prepared according to the invention can be cultured continuously or discontinuously in the batch process (batch culture) or in the fed batch (feed process) or repeated fed batch process (repetitive feed process) for the purpose of L-lysine production. A summary of known culture methods is described in the textbook by Chmiel (Bioprozesstechnik 1. Einführung in die Bioverfahrenstechnik [Bioprocess Technology 1. Introduction to Bioprocess Technology (Gustav Fischer Verlag, Stuttgart, 1991)) or in the textbook by Storhas (Bioreaktoren und periphere Einrichtungen [Bioreactors and Peripheral Equipment] (Vieweg Verlag, Braunschweig/Wiesbaden, 1994)). The culture medium to be used must meet the requirements of the particular microorganisms in a suitable manner. Descriptions of culture media for various microorganisms are contained in the handbook "Manual of Methods for General Bacteriology" of the American Society for Bacteriology (Washington D.C., USA, 1981). Sugars and carbohydrates, such as e.g. glucose, sucrose, lactose, fructose, maltose, molasses, starch and cellulose, oils and fats, such as e. g. soya oil, sunflower oil, groundnut oil and coconut fat, fatty acids, such as e. g. palmitic acid, stearic acid and linoleic acid, alcohols, such as e. g. glycerol and ethanol, and organic acids, such as e. g. acetic acid, can be used as the source of carbon. These substance can be used individually or as a mixture. Organic nitrogen-containing compounds, such as peptones, yeast extract, meat extract, malt extract, corn steep liquor, soya bean flour and urea, or inorganic compounds, such as ammonium sulphate, ammonium chloride, ammonium phosphate, ammonium carbonate and ammonium nitrate, can be used as the source of nitrogen. The sources of nitrogen can be used individually or as a mixture. Potassium dihydrogen phosphate or dipotassium hydrogen phosphate or the corresponding sodium-containing salts can be used as the source of phosphorus. The culture medium must furthermore comprise salts of metals, such as e. g. magnesium sulfate or iron sulfate, which are necessary for growth. Finally, essential growth substances, such as amino acids and vitamins, can be employed in addition to the above-mentioned substances. Suitable precursors can moreover be added to the culture medium. The starting substances mentioned can be added to the culture in the form of a single batch, or can be fed in during the culture in a suitable manner.

Basic compounds, such as sodium hydroxide, potassium hydroxide, ammonia, or acid compounds, such as phosphoric acid or sulfuric acid, can be employed in a suitable manner to control the pH. Antifoams, such as e.g. fatty acid polyglycol esters, can be employed to control the development of foam. Suitable substances having a selective action, e.g. antibiotics, can be added to the medium to maintain the stability of plasmids. To maintain aerobic conditions, oxygen or oxygen-containing gas mixtures, such as e.g. air, are introduced into the culture. The temperature of the culture is usually 20°C to 45°C, and preferably 25°C to 40°C. Culturing is continued until a maximum of L-lysine has formed. This target is usually reached within 10 hours to 160 hours.

The analysis of L-lysine can be carried out by anion exchange chromatography with subsequent ninhydrin derivatization, as described by Spackman et al. (Analytical Chemistry, 30, (1958), 1190), or it can take place by reversed phase HPLC as described by Lindroth et al. (Analytical Chemistry (1979) 51:. 1167-1174).

The following microorganism has been deposited at the Deutsche Sammlung für Mikroorganismen und Zellkulturen (DSMZ = German Collection of Microorganisms and Cell Cultures, Braunschweig, Germany) in accordance with the Budapest Treaty:

Escherichia coli K-12 DH5α/pEC-T18mob2 as DSM 13244

The following figures are attached:
- Figure 1: Map of the plasmid pEC-T18mob2
- Figure 2: Map of the plasmid pMS82
- Figure 3: Map of the plasmid pMS82B
- Figure 4: Map of the plasmid pCR2.1poxBint

The base pair numbers stated are approx. values obtained in the context of reproducibility.

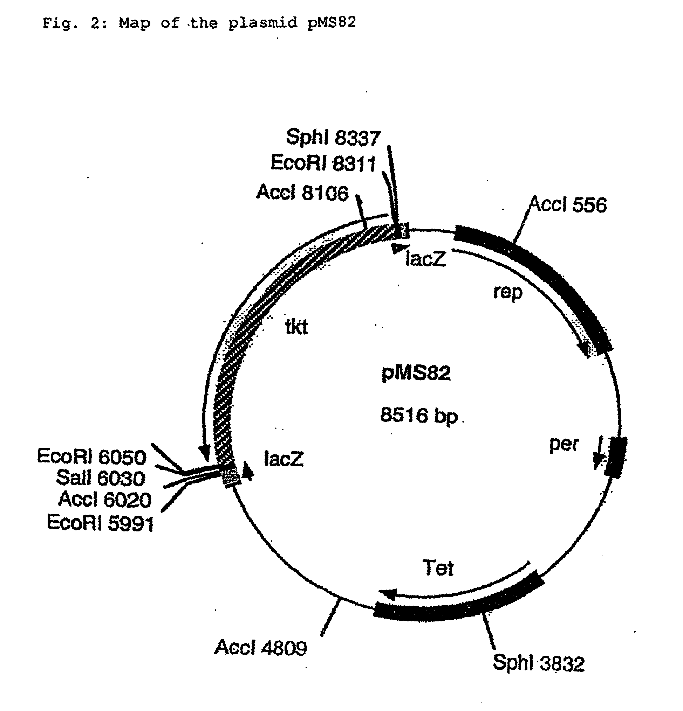

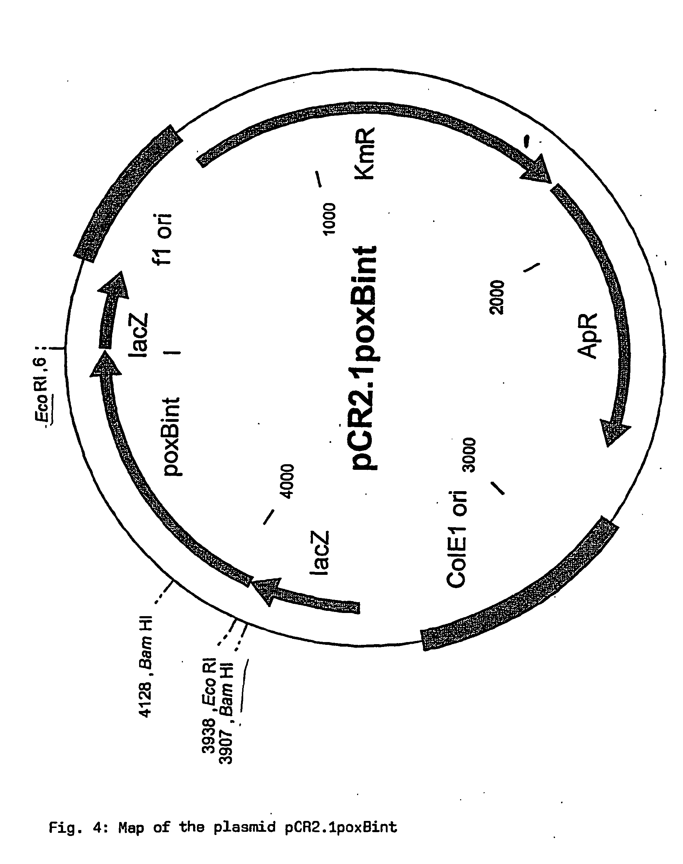

The abbreviations used have the following meaning:
Re Figure 1:
   - Tet:: Resistance gene for tetracycline
   - oriV:: Plasmid-coded replication origin of E. coli
   - RP4mob:: mob region for mobilizing the plasmid
   - rep:: Plasmid-coded replication origin from C. glutamicum plasmid pGA1
   - per:: Gene for controlling the number of copies from pGA1
   - lacZ-alpha:: lacZα gene fragment (N-terminus) of the β-galactosidase gene
Re Figure 2 and 3:
   - Tet:: Resistance gene for tetracycline
   - rep:: Plasmid-coded replication origin from C. glutamicum plasmid pGA1
   - per:: Gene for controlling the number of copies from PGA1
   - lacZ: Cloning relict of the lacZα gene fragment from pEC-T18mob2
   - tkt:: Transketolase gene
Re Figure 4:
   - ColE1 ori:: Replication origin of the plasmid ColE1
   - lacZ:: Cloning relict of the lacZα gene fragment
   - fl ori:: Replication origin of phage f1
   - KmR:: Kanamycin resistance
   - ApR:: Ampicillin resistance
   - poxBint:: internal fragment of the poxB gene

Moreover, the following abbreviations have been used:
- AccI:: Cleavage site of the restriction enzyme AccI
- BamHI:: Cleavage site of the restriction enzyme BamHI
- EcoRI:: Cleavage site of the restriction enzyme EcoRI
- HindIII:: Cleavage site of the restriction enzyme HindIII
- KpnI:: Cleavage site of the restriction enzyme KpnI
- PstI:: Cleavage site of the restriction enzyme PstI
- PvuI:: Cleavage site of the restriction enzyme PvuI
- SalI:: Cleavage site of the restriction enzyme SalI
- SacI:: Cleavage site of the restriction enzyme SacI
- SmaI:: Cleavage site of the restriction enzyme SmaI
- SphI:: Cleavage site of the restriction enzyme SphI
- XbaI:: Cleavage site of the restriction enzyme XbaI
- XhoI:: Cleavage site of the restriction enzyme XhoI

### Examples

The following examples will further illustrate this invention. The molecular biology techniques, e.g. plasmid DNA isolation, restriction enzyme treatment, ligations, standard transformations of Escherichia coli etc. used are, (unless stated otherwise), described by Sambrook et al., (Molecular Cloning. A Laboratory Manual (1989) Cold Spring Harbour Laboratories, USA).

### Example 1

### Construction of a gene library of Corynebacterium glutamicum strain AS019

A DNA library of Corynebacterium glutamicum strain ASO19 (Yoshihama et al., Journal of Bacteriology 162, 591-597 (1985)) was constructed using λ Zap Express^{™} system, (Short et al., (1988) Nucleic Acids Research, 16: 7583-7600), as described by O'Donohue (O'Donohue, M. (1997). The Cloning and Molecular Analysis of Four Common Aromatic Amino Acid Biosynthetic Genes from Corynebacterium glutamicum. Ph.D. Thesis, National University of Ireland, Galway.). λ Zap Express^{™} kit was purchased from Stratagene (Stratagene, 11011 North Torrey Pines Rd., La Jolla, California 92037.) and used according to the manufacturers instructions. AS019-DNA was digested with restriction enzyme Sau3A and ligated to BamHI treated and dephosphorylated λ Zap Express^{™} arms.

### Example 2

### Cloning and sequencing of the tkt gene

### 1. Cloning

An Escherichia coli strain, AI1118, carrying mutations in the tktA and tktB genes as described by Iida et al., 1993 (Identification and characterization of the tktB gene encoding a second transketolase in Escherichia coli K-12. Journal of Bacteriology 175: 5375-83), was transformed with approx. 500 ng of the AS019 λ Zap Express^{™} plasmid library described above. Selection for transformants was made on M9 minimal media, (Sambrook et al (1989). Molecular Cloning. A Laboratory Manual Cold Spring Harbour Laboratories, USA), containing kanamycin at a concentration of 50 mg/l and incubation at 37°C for 48 hours. Plasmid DNA was isolated from one transformant as according to Birnboim and Doly, 1979, (A rapid alkaline extraction procedure for screening recombinant plasmid DNA. Nucleic Acids Research, 7: 1513-1523.), and designated pTSM2.

### 3. Sequencing

The clone pTSM2 was commercially sequenced by MWG-Biotech Ltd., Waterside House, Peartree Bridge, Milton Keynes MK6 3BY, U.K. High purity plasmid DNA was prepared for MWG-Biotech, using the QIAprep Spin Miniprep Kit (QIAGEN GmbH, Max-Volmer-Strasse 4, 40724 Hilden, Germany), and subsequently freeze dried using a Lyovac GT 2 freeze dryer (Leybold Heraeus). Initial sequence analysis was carried out using the universal forward and M13 reverse primers.
M13/pUC forward primer: 5' GTAAAACGACGGCCAGT 3'
M13/pUC reverse primer: 5' CAGGAAACAGCTATGAC 3'

An internal primer was subsequently designed from the sequence obtained which allowed the entire tkt gene to be deduced. The sequence of the internal primer was as follows:
Internal primer 1:
5' TGCAGCAACCAAGACTG 3'

Sequence obtained was then analyzed using the DNA Strider programme, (Marck (1988), Nucleic Acids Research 16: 1829-1836), version 1.0 on an Apple Macintosh computer. This program allowed for analyses such as restriction site usage, open reading frame analysis and codon usage determination. Searches between DNA sequence obtained and those in EMBL and Genbank databases were achieved using the BLAST programme .(Altschul et al., (1997). Nucleic Acids Research, 25: 3389-3402). DNA and protein sequences were aligned using the Clustal V and Clustal W programs (Higgins and Sharp, 1988 Gene 73: 237-244).

The sequence thus obtained is shown in SEQ ID NO 1. The analysis of the nucleotide sequence obtained revealed an open reading frame of 2094 base pairs which was designated as tkt gene. It codes for a protein of 697 amino acids shown in SEQ ID NO 2.

### Example 3

### Expression of the tkt gene

### 3.1 Preparation of the shuttle vector pEC-T18mob2

The E. coli - C. glutamicum shuttle vector pEC-T18mob2 was constructed according to the prior art.

The vector contains the replication region rep of the plasmid pGA1 including the replication effector per (US-A-5,175,108; Nesvera et al., Journal of Bacteriology 179, 1525-1532 (1997)), the tetracycline resistance-imparting tetA(Z) gene of the plasmid pAG1 (US-A- 5,158,891; gene library entry at the National Center for Biotechnology Information (NCBI, Bethesda, MD, USA) with accession number AF121000), the replication region oriV of the plasmid pMB1 (Sutcliffe, Cold Spring Harbor Symposium on Quantitative Biology 43, 77-90 (1979)), the lacZα gene fragment including the lac promoter and a multiple cloning site (mcs) (Norrander et al. Gene 26, 101-106 (1983)) and the mob region of the plasmid RP4 (Simon et al., (1983) Bio/Technology 1:784-791).

The vector constructed was transformed in the E. coli strain DH5α (Hanahan, In: DNA cloning. A practical approach. Vol. I. IRL-Press, Oxford, Washington DC, USA). Selection for plasmid-carrying cells was made by plating out the transformation batch on LB agar (Sambrook et al., Molecular cloning: a laboratory manual. 2nd Ed. Cold Spring Harbor Laboratory Press, Cold Spring Harbor, N.Y.), which had been supplemented with 5 mg/l tetracycline. Plasmid DNA was isolated from a transformant with the aid of the QIAprep Spin Miniprep Kit from Qiagen and checked by restriction with the restriction enzymes EcoRI and HindIII subsequent agarose gel electrophoresis (0.8%).

The plasmid was called pEC-T18mob2 and is shown in Figure 1. It is deposited in the form of the strain Escherichia coli K-12 strain DH5α/pEC-T18mob2 at the Deutsche Sammlung für Mikroorganismen und Zellkulturen (DSMZ = German Collection of Microorganisms and Cell Cultures, Braunschweig, Germany) as DSM 13244.

### 3.2 Cloning of the tkt gene into the E. coli - C. glutamicum shuttle vector pEC-T18mob2

PCR was used to amplify DNA fragments containing the entire tkt gene of C. glutamicum and flanking upstream and downstream regions. PCR reactions were carried out using oligonucleotide primers designed from SEQ ID NO 1. Genomic DNA was isolated from Corynebacterium glutamicum ATCC13032 according to Heery and Dunican, (Applied and Environmental Microbiology 59: 791-799 (1993))and approx. 150-200 ng used as template. The primers used were:
tkt fwd. primer: 5' CTG ATC ATC GGA TCT AAC GAA 3'
tkt rev. primer: 5' ATT GCC CCG GGT TGA AGC TAA3 3'

PCR parameters were as follows:
35 cycles
95°C for 6 minutes
94°C for 1 minute
55°C for 1 minute
72°C for 45 seconds
1 mM MgCl₂

The PCR product obtained was cloned into the commercially available pGEM-T vector purchased from Promega Corp. (pGEM-T Easy Vector System 1, cat. no. A1360, Promega UK, Southampton) using E. coli strain JM109 (Yanisch-Perron et al., Gene 33: 103-119 (1985)) as a host. The entire tkt gene was subsequently isolated from the pGEM T-vector on an SphI/SalI fragment and cloned into the lacZ SphI/SalI region of the E. coli - C. glutamicum shuttle vector pEC-T18mob2 (Figure 1), and designated pMS82 (Figure 2). Restriction enzyme analysis with AccI (Boehringer Mannheim GmbH, Germany) revealed the incorrect orientation of the tkt gene in the lacZα gene of pEC-T18mob2. The orientation was corrected by restricting with EcoRI enzyme (Boehringher Mannheim GmbH, Germany) and religating. Restriction enzyme analysis with AccI (Boehringer Mannheim GmbH, Germany) revealed the correct orientation of the tkt gene in the lacZα gene (i. e. downstream the lac-Promotor) of pEC-T18mob2 and this plasmid was designated the name pMS82B (Figure 3).

### Example 4

### Effect of over-expression of the tkt gene in various lysine producers

The L-lysine-producing strain Corynebacterium glutamicum DSM5715 is described in EP-B-0435132 and the strain DSM12866 is described in DE-A-19931314.8. Both strains are deposited at the Deutsche Sammlung für Mikroorganismen und Zellkulturen [German Collection of Microorganisms and Cell Cultures] in Braunschweig (Germany) in accordance with the Budapest Treaty.

### 4.1 Preparation of the strains DSM5715/pMS82B and DSM12866/pMS82B

The strains DSM5715 and DSM12866 were transformed with the plasmid pMS82B using the electroporation method described by Liebl et al. (FEMS Microbiology Letters, 53:299-303 (1989)). Selection of the transformants took place on LBHIS agar comprising 18.5 g/l brain-heart infusion broth, 0.5 M sorbitol, 5 g/l Bacto-tryptone, 2.5 g/l Bacto-yeast extract, 5 g/l NaC1 and 18 g/l Bacto-agar, which had been supplemented with 5 mg/l tetracycline. Incubation was carried out for 2 days at 33°C.

Plasmid DNA was isolated in each case from a transformant by conventional methods (Peters-Wendisch et al., 1998, Microbiology, 144, 915 - 927), cleaved with the restriction endonuclease AccI, and the plasmid was checked by subsequent agarose gel electrophoresis. The strains obtained in this way were called DSM5715/pMS82B and DSM12866/pMS82B.

### 4.2 Preparation of L-lysine

The Corynebacterium glutamicum strains DSM5715/pMS82B and DSM12866/pMS82B obtained in Example 4.1 were cultured in a nutrient medium suitable for the production of lysine and the lysine content in the culture supernatant was determined.

For this, the strain was first incubated on an agar plate with the corresponding antibiotic (brain-heart agar with tetracycline (5 mg/1)) for 24 hours at 33°C. Starting from this agar plate culture, a preculture was seeded (10 ml medium in a 100 ml conical flask). The complete medium CgIII was used as the medium for the preculture.

| | |
|---|---|
| Medium Cg III | |
| NaCl | 2.5 g/l |
| Bacto-Peptone | 10 g/l |
| Bacto-Yeast extract | 10 g/l |
| Glucose (autoclaved separately) | 2% (w/v) |

### The pH was brought to pH 7.4

Tetracycline (5 mg/1) was added to this. The preculture was incubated for 16 hours at 33°C at 240 rpm on a shaking machine. A main culture was seeded from this preculture such that the initial OD (660nm) of the main culture was 0.1. Medium MM was used for the main culture.

| | |
|---|---|
| Medium MM | |
| CSL (corn steep liquor) | 5 g/l |
| MOPS (morpholinopropanesulfonic acid) | 20 g/l |
| Glucose (autoclaved separately) | 50 g/l |
| | |
| (NH₄)₂SO₄ | 25 g/l |
| KH₂PO₄ | 0.1 g/l |
| MgSO₄ * 7 H₂O | 1.0 g/l |
| CaCl₂ * 2 H₂O | 10 mg/l |
| FeSO₄ * 7 H₂O | 10 mg/l |
| MnSO₄ * H₂O | 5.0mg/l |
| Biotin (sterile-filtered) | 0.3 mg/l |
| Thiamine * HCl (sterile-filtered) | 0.2 mg/l |
| L-Leucine (sterile-filtered) | 0.1 g/l |
| CaCO₃ | 25 g/l |

The CSL, MOPS and the salt solution were brought to pH 7 with aqueous ammonia and autoclaved. The sterile substrate and vitamin solutions were then added, as well as the CaCO₃ autoclaved in the dry state.

Culturing is carried out in a 10 ml volume in a 100 ml conical flask with baffles. Tetracycline (5 mg/1) was added. Culturing was carried out at 33°C and 80% atmospheric humidity.

After 72 hours, the OD was determined at a measurement wavelength of 660 nm with a Biomek 1000 (Beckmann Instruments GmbH, Munich). The amount of lysine formed was determined with an amino acid analyzer from Eppendorf-BioTronik (Hamburg, Germany) by ion exchange chromatography and post-column derivatization with ninhydrin detection.

The result of the experiment is shown in Table 1.

**Table 1**

| Strain | OD | L-Lysine HCl g/l |
|---|---|---|
| DSM5715 | 7.2 | 14.1 |
| DSM5715/pMS82B | 7.2 | 14.8 |
| DSM12866 | 10.9 | 15.3 |
| DSM12866/pMS82B | 11.2 | 16.8 |

### Example 5

### Preparation of a genomic cosmid gene library from Corynebacterium glutamicum ATCC 13032

Chromosomal DNA from Corynebacterium glutamicum ATCC 13032 was isolated as described by Tauch et al., (1995, Plasmid 33:168-179), and partly cleaved with the restriction enzyme Sau3AI (Amersham Pharmacia, Freiburg, Germany, Product Description Sau3AI, Code no. 27-0913-02). The DNA fragments were dephosphorylated with shrimp alkaline phosphatase (Roche Molecular Biochemicals, Mannheim, Germany, Product Description SAP, Code no. 1758250). The DNA of the cosmid vector SuperCosl (Wahl et al. (1987) Proceedings of the National Academy of Sciences USA 84:2160-2164), obtained from Stratagene (La Jolla, USA, Product Description SuperCosl Cosmid Vektor Kit, Code no. 251301) was cleaved with the restriction enzyme XbaI (Amersham Pharmacia, Freiburg, Germany, Product Description XbaI, Code no. 27-0948-02) and likewise dephosphorylated with shrimp alkaline phosphatase. The cosmid DNA was then cleaved with the restriction enzyme BamHI (Amersham Pharmacia, Freiburg, Germany, Product Description BamHI, Code no. 27-0868-04). The cosmid DNA treated in this manner was mixed with the treated ATCC13032 DNA and the batch was treated with T4 DNA ligase (Amersham Pharmacia, Freiburg, Germany, Product Description T4-DNA-Ligase, Code no.27-0870-04). The ligation mixture was then packed in phages with the aid of Gigapack II XL Packing Extracts (Stratagene, La Jolla, USA, Product Description Gigapack II XL Packing Extract, Code no. 200217). For infection of the E. coli strain NM554 (Raleigh et al. 1988, Nucleic Acid Res. 16:1563-1575) the cells were taken up in 10 mM MgSO₄ and mixed with an aliquot of the phage suspension. The infection and titering of the cosmid library were carried out as described by Sambrook et al. (1989, Molecular Cloning: A laboratory Manual, Cold Spring Harbor), the cells being plated out on LB agar (Lennox, 1955, Virology, 1:190) + 100 µg/ml ampicillin. After incubation overnight at 37°C, recombinant individual clones were selected.

### Example 6

### Isolation and sequencing of the poxB gene

The cosmid DNA of an individual colony (Example 5) was isolated with the Qiaprep Spin Miniprep Kit (Product No. 27106, Qiagen, Hilden, Germany) in accordance with the manufacturer's instructions and partly cleaved with the restriction enzyme Sau3AI (Amersham Pharmacia, Freiburg, Germany, Product Description Sau3AI, Product No. 27-0913-02). The DNA fragments were dephosphorylated with shrimp alkaline phosphatase (Roche Molecular Biochemicals, Mannheim, Germany, Product Description SAP, Product No. 1758250). After separation by gel electrophoresis, the cosmid fragments in the size range of 1500 to 2000 bp were isolated with the QiaExII Gel Extraction Kit (Product No. 20021, Qiagen, Hilden, Germany). The DNA of the sequencing vector pZero-1, obtained from Invitrogen (Groningen, Holland, Product Description Zero Background Cloning Kit, Product No. K2500-01) was cleaved with the restriction enzyme BamHI (Amersham Pharmacia, Freiburg, Germany, Product Description BamHI, Product No. 27-0868-04). The ligation of the cosmid fragments in the sequencing vector pZero-1 was carried out as described by Sambrook et al. (1989, Molecular Cloning: A laboratory Manual, Cold Spring Harbor), the DNA mixture being incubated overnight with T4 ligase (Pharmacia Biotech, Freiburg, Germany). This ligation mixture was then electroporated (Tauch et al. 1994, FEMS Microbiol Letters, 123:343-7) into the E. coli strain DH5αMCR (Grant, 1990, Proceedings of the National Academy of Sciences U.S.A. 87:4645-4649) and plated out on LB agar (Lennox, 1955, Virology, 1:190) with 50 µg/ml zeocin. The plasmid preparation of the recombinant clones was carried out with Biorobot 9600 (Product No. 900200, Qiagen, Hilden, Germany). The sequencing was carried out by the dideoxy chain-stopping method of Sanger et al. (1977, Proceedings of the National Academies of Sciences U.S.A., 74:5463-5467) with modifications according to Zimmermann et al. (1990, Nucleic Acids Research 18:1067). The "RR dRhodamin Terminator Cycle Sequencing Kit" from PE Applied Biosystems(Product No. 403044, Weiterstadt, Germany) was used. The separation by gel electrophoresis and analysis of the sequencing reaction were carried out in a "Rotiphoresis NF Acrylamide/Bisacrylamide" Gel (29:1) (Product No. A124.1, Roth, Karlsruhe, Germany) with the "ABI Prism 377" sequencer from PE Applied Biosystems (Weiterstadt, Germany).

The raw sequence data obtained were then processed using the Staden program package (1986, Nucleic Acids Research, 14:217-231) version 97-0. The individual sequences of the pZerol derivatives were assembled to a continuous contig. The computer-assisted coding region analysis was prepared with the XNIP program (Staden, 1986, Nucleic Acids Research 14:217-231). Further analyses were carried out with the "BLAST search programs" (Altschul et al., 1997, Nucleic Acids Research, 25:3389-3402), against the non-redundant databank of the "National Center for Biotechnology Information" (NCBI, Bethesda, MD, USA).

The resulting nucleotide sequence is shown in SEQ ID No. 3. Analysis of the nucleotide sequence showed an open reading frame of 1737 base pairs, which was called the poxB gene. The poxB gene codes for a polypeptide of 579 amino acids (SEQ ID NO. 4).

### Example 7

### Preparation of an integration vector for integration mutagenesis of the poxB gene

From the strain ATCC 13032, chromosomal DNA was isolated by the method of Eikmanns et al. (Microbiology 140: 1817 - 1828 (1994)). On the basis of the sequence of the poxB gene known for C. glutamicum from Example 8, the following oligonucleotides were chosen for the polymerase chain reaction:
poxBint1:
   5' TGC GAG ATG GTG AAT GGT GG 3'
poxBint2:
   5` GCA TGA GGC AAC GCA TTA GC 3`

The primers shown were synthesized by MWG Biotech (Ebersberg, Germany) and the PCR reaction was carried out by the standard PCR method of Innis et al. (PCR protocols. A guide to methods and applications, 1990, Academic Press) with Pwo-Polymerase from Boehringer. With the aid of the polymerase chain reaction, a DNA fragment approx. 0.9 kb in size was isolated, this carrying an internal fragment of the poxB gene and being shown in SEQ ID No. 5.

The amplified DNA fragment was ligated with the TOPO TA Cloning Kit from Invitrogen Corporation (Carlsbad, CA, USA; Catalogue Number K4500-01) in the vector pCR2.1-TOPO (Mead at al. (1991) Bio/Technology 9:657-663). The E. coli strain DH5α was then electroporated with the ligation batch (Hanahan, In: DNA cloning. A practical approach. Vol.I. IRL-Press, Oxford, Washington DC, USA, 1985). Selection for plasmid-carrying cells was made by plating out the transformation batch on LB agar (Sambrook et al., Molecular cloning: a laboratory manual. 2nd Ed. Cold Spring Harbor Laboratory Press, Cold Spring Harbor, N.Y., 1989), which had been supplemented with 25 mg/l kanamycin. Plasmid DNA was isolated from a transformant with the aid of the QIAprep Spin Miniprep Kit from Qiagen and checked by restriction with the restriction enzyme EcoRI and subsequent agarose gel electrophoresis (0.8%). The plasmid was called pCR2.1poxBint (Figure 4).

Plasmid pCR2.1poxBint has been deposited in the form of the strain Escherichia coli DH5α/pCR2. 1poxBint as DSM 13114 at the Deutsche Sammlung für Mikroorganismen und Zellkulturen (DSMZ = German Collection of Microorganisms and Cell Cultures, Braunschweig, Germany) in accordance with the Budapest Treaty.

### Example 8

### Integration mutagenesis of the poxB gene in the lysine producer DSM 5715

The vector pCR2.1poxBint mentioned in Example 7 was electroporated by the electroporation method of Tauch et al.(FEMS Microbiological Letters, 123:343-347 (1994)) in Corynebacterium glutamicum DSM 5715. Strain DSM 5715 is an AEC-resistant lysine producer. The vector pCR2.1poxBint cannot replicate independently in DSM5715 and is retained in the cell only if it has integrated into the chromosome of DSM 5715. Selection of clones with pCR2.1poxBint integrated into the chromosome was carried out by plating out the electroporation batch on LB agar (Sambrook et al., Molecular cloning: a laboratory manual. 2nd Ed. Cold Spring Harbor Laboratory Press, Cold Spring Harbor, N.Y.), which had been supplemented with 15 mg/l kanamycin. For detection of the integration, the poxBint fragment was labelled with the Dig hybridization kit from Boehringer by the method of "The DIG System Users Guide for Filter Hybridization" of Boehringer Mannheim GmbH (Mannheim, Germany, 1993). Chromosomal DNA of a potential integrant was isolated by the method of Eikmanns et al. (Microbiology 140: 1817 - 1828 (1994)) and in each case cleaved with the restriction enzymes SalI, SacI and HindIII. The fragments formed were separated by agarose gel electrophoresis and hybridized at 68°C with the Dig hybrization kit from Boehringer. The plasmid pCR2.1poxBint mentioned in Example 9 had been inserted into the chromosome of DSM5715 within the chromosomal poxB gene. The strain was called DSM5715::pCR2.1poxBint.

### Example 9

Effect of over-expression of the tkt gene with simultaneous elimination of the poxB gene on the preparation of lysine

### 9.1 Preparation of the strain DSM5715::pCR2.1poxBint/pMS82B

The strain DSM5715::pCR2.1poxBint was transformed with the plasmid pMS82B using the electroporation method described by Liebl et al. (FEMS Microbiology Letters, 53:299-303 (1989)). Selection of the transformants took place on LBHIS agar comprising 18.5 g/l brain-heart infusion broth, 0.5 M sorbitol, 5 g/l Bacto-tryptone, 2.5 g/l Bacto-yeast extract, 5 g/l NaCl and 18 g/l Bacto-agar, which had been supplemented with 5 mg/l tetracycline and 25 mg/l kanamycin. Incubation was carried out for 2 days at 33°C.

Plasmid DNA was isolated in each case from a transformant by conventional methods (Peters-Wendisch et al., 1998, Microbiology 144, 915 -927), cleaved with the restriction endonuclease AccI, and the plasmid was checked by subsequent agarose gel electrophoresis. The strain obtained in this way was called DSM5715:pCR2.1poxBint/pMS82B.

### 9.2 Preparation of L-lysine

The C. glutamicum strain DSM5715::pCR2.1poxBint/pMS82B obtained in Example 9.1 was cultured in a nutrient medium suitable for the production of lysine and the lysine content in the culture supernatant was determined.

For this, the strain was first incubated on an agar plate with the corresponding antibiotic (brain-heart agar with tetracycline (5 mg/l) and kanamycin (25 mg/l)) for 24 hours at 33°C. Starting from this agar plate culture, a preculture was seeded (10 ml medium in a 100 ml conical flask). The complete medium CgIII was used as the medium for the preculture.

| Medium Cg III | |
|---|---|
| NaCl | 2.5 g/l |
| Bacto-Peptone | 10 g/l |
| Bacto-Yeast extract | 10 g/l |
| Glucose (autoclaved separately) | 2% (w/v) |

The pH was brought to pH 7.4

Tetracycline (5 mg/1) and kanamycin (25 mg/1) were added to this. The preculture was incubated for 16 hours at 33°C at 240 rpm on a shaking machine. A main culture was seeded from this preculture such that the initial OD (660nm) of the main culture was 0.1. Medium MM was used for the main culture.

| Medium MM | |
|---|---|
| CSL (corn steep liquor) | 5 g/l |
| MOPS (morpholinopropanesulfonic acid) | 20 g/l |
| Glucose (autoclaved separately) | 58 g/l |
| | |
| (NH₄)₂SO₄ | 25 g/l |
| KH₂PO₄ | 0.1 g/l |
| MgSO₄ * 7 H₂O | 1.0 g/l |
| CaCl₂ * 2 H₂O | 10 mg/l |
| FeSO₄ * 7 H₂O | 10 mg/l |
| MnSO₄ * H₂O | 5.0mg/l |
| Biotin (sterile-filtered) | 0.3 mg/l |
| Thiamine * HCl (sterile-filtered) | 0.2 mg/l |
| L-Leucine (sterile-filtered) | 0.1 g/l |
| CaCO₃ | 25 g/l |

The CSL, MOPS and the salt solution were brought to pH 7 with aqueous ammonia and autoclaved. The sterile substrate and vitamin solutions were then added, as well as the CaCO₃ autoclaved in the dry state.

Culturing is carried out in a 10 ml volume in a 100 ml conical flask with baffles. Tetracycline (5 mg/l) and kanamycin (25 mg/l) were added. Culturing was carried out at 33°C and 80% atmospheric humidity.

After 72 hours, the OD was determined at a measurement wavelength of 660 nm with a Biomek 1000 (Beckmann Instruments GmbH, Munich). The amount of lysine formed was determined with an amino acid analyzer from Eppendorf-BioTronik (Hamburg, Germany) by ion exchange chromatography and post-column derivatization with ninhydrin detection.

The result of the experiment is shown in Table 2.

**Table 2**

| Strain | OD | L-Lysine FiCl g/l |
|---|---|---|
| DSM5715 | 10.8 | 15.9 |
| DSM5715::pCR2.1poxBint | 7.1 | 16.7 |
| DSM5715::pCR2.1poxBint/ pMS82B | 7.7 | 17.3 |

## Claims

1. A process for the preparation of L-Lysine, using a coryneform bacterium producing L-Lysine which comprises carrying out the following steps:
a) amplification of at least the tkt gene coding for transketolase in said coryneform bacterium,
b) fermentation of the bacterium of a) in a medium,
c) concentration of L-Lysine in the medium or in the cells of the coryneform bacterium, and
d) isolation of L-Lysine produced,
wherein, during said fermentation, the concentration of L-Lysine increases above the level obtained with a coryneform bacterium, in which the tkt gene is not amplified.

2. A process as claimed in claim 1, wherein in the coryneform bacterium one or more genes chosen from the group consisting of
2.1 the dapA gene which codes for dihydrodipicolinate synthase
2.2 the zwf gene which codes for glucose 6-phosphate dehydrogenase,
2.3 the gnd gene which codes for 6-phosphogluconate dehydrogenase,
2.4 the eno gene which codes for enolase
is or are amplified or over-expressed at the same time.

3. A process as claimed in claim 1, wherein in the coryneform bacterium one or more genes chosen from the group consisting of,
3.1 the pck gene which codes for phosphoenol pyruvate carboxykinase,
3.2. the pgi gene which codes for glucose 6-phosphate isomerase,
3.3 the poxB gene which codes for pyruvate oxidase, is or are attenuated at the same time.

4. A process as claimed in claims 1 to 2, wherein to achieve the amplification, the number of copies of the genes or nucleotide sequences is increased by transformation of the microorganisms with plasmid vectors which carry these genes or nucleotide sequences.

## Patentansprüche

1. Verfahren zur Herstellung von L-Lysin unter Verwendung eines L-Lysin produzierenden coryneformen Bakteriums, wobei man die folgenden Schritte durchführt:
a) Verstärkung zumindest des für die Transketolase codierenden tkt-Gens in dem coryneformen Bakterium,
b) Fermentation des Bakteriums aus a) in einem Medium,
c) Aufkonzentrierung von L-Lysin im Medium oder in den Zellen des coryneformen Bakteriums und
d) Isolierung des produzierten L-Lysins,
wobei während der Fermentation die Konzentration des L-Lysins über das mit einem coryneformen Bakterium, in dem das tkt-Gen nicht verstärkt ist, erhaltene Niveau ansteigt.

2. Verfahren nach Anspruch 1, wobei in dem coryneformen Bakterium ein oder mehrere Gene, gewählt aus der Gruppe
2.1 das für die Dihydrodipicolinat-Synthase codierende dapA-Gen,
2.2 das für die Glucose-6-phosphat-Dehydrogenase codierende zwf-Gen,
2.3 das für die 6-Phosphogluconat-Dehydrogenase codierende gnd-Gen,
2.4 das für die Enolase codierende eno-Gen,
gleichzeitig verstärkt oder überexprimiert wird bzw. werden.

3. Verfahren nach Anspruch 1, wobei in dem coryneformen Bakterium ein oder mehrere Gene, gewählt aus der Gruppe
3.1 das für die Phosphoenolpyruvat-Carboxykinase codierende pck-Gen,
3.2 das für die Glucose-6-phosphat-Isomerase codierende pgi-Gen,
3.3 das für die Pyruvat-Oxidase codierende poxB-Gen,
gleichzeitig abgeschwächt wird bzw. werden.

4. Verfahren nach Anspruch 1 und 2, wobei zur Erzielung der Verstärkung die Kopienzahl der Gene oder Nukleotidsequenzen durch Transformation der Mikroorganismen mit Plasmidvektoren, die diese Gene bzw. Nukleotidsequenzen tragen, erhöht wird.

## Revendications

1. Procédé pour la préparation de L-lysine utilisant une bactérie coryneforme productrice de L-lysine qui comprend les opérations suivantes :
a) amplification d'au moins le gène tkt codant pour la transcétolase dans ladite bactérie coryneforme,
b) fermentation de la bactérie de a) dans un milieu,
c) concentration de la L-lysine dans le milieu ou dans des cellules de la bactérie coryneforme, et
d) isolation de la L-lysine produite,
dans lequel, pendant ladite fermentation, la concentration de la L-lysine s'élève au-delà du niveau obtenu avec une bactérie coryneforme, dans laquelle le gène tkt n'est pas amplifié.

2. Procédé selon la revendication 1, dans lequel, dans la bactérie coryneforme, un ou plusieurs gènes choisis dans le groupe constitué de :
2.1 le gène dapA qui code pour la dihydrodipicolinate synthase
2.2 le gène zwf qui code pour la glucose 6-phosphate déshydrogénase,
2.3 le gène gnd qui code pour la 6-phosphogluconate déshydrogénase,
2. 4 le gène eno qui code pour l'énolase
est ou sont amplifiés ou surexprimés simultanément.

3. Procédé selon la revendication 1, dans lequel, dans la bactérie coryneforme, un ou plusieurs gènes choisis dans le groupe constitué de :
3.1 le gène pck qui code pour la phosphoénol pyruvate carboxykinase,
3.2 le gène pgi qui code pour la glucose 6-phosphate isomérase,
3.3 le gène poxB qui code pour la pyruvate oxydase,
est ou sont atténués simultanément.

4. Procédé selon la revendication 1 ou 2, dans lequel pour obtenir l'amplification, le nombre de copies des gènes ou des séquences nucléotidiques est accru par transformation de microorganismes avec des vecteurs plasmidiques qui sont porteurs de ces gènes ou séquences nucléotidiques.


### SEQUENCE PROTOCOL

<110> National University of Ireland, Galway Degussa-Htlls AG
<120> Process for the fermentative preparation of L-amino acids using coryneform bacteria.
<130> 000019 BT
<140>
   <141>
<160> 5
<170> PatentIn Ver. 2.1
<210> 1
   <211> 2350
   <212> DNA
   <213> Corynebacterium glutamicum
<220>
   <221> CDS
   <222> (205)..(2295)
   <223> tkt
<400> 1
   cgggtcagat taagcaaaga ctactttcgg ggtagatcac ctttgccaaa tttgaaccaa 60
   ttaacctaag tcgtagatct gatcatcgga tctaacgaaa acgaaccaaa actttggtcc 120
   cggtttaacc caggaaggat tgaccaccta acgaaaacga accaaaactt tggtcccggt 180
   ttaacccagg aaggattgac cacc ttg acg ctg tca cct gaa ctt cag gcg   231
                              Leu Thr Leu Ser Pro Glu Leu Gln Ala
                               1               5
   ctc act gta cgc aat tac ccc tct gat tgg tcc gat gtg gac acc aag   279
   Leu Thr Val Arg Asn Tyr Pro Ser Asp Trp Ser Asp Val Asp Thr Lys
    10                  15                  20                  25
   gct gta gac act gtt cgt gtc ctc gct gca gac gct gta gaa aac tgt   327
   Ala Val Asp Thr Val Arg Val Leu Ala Ala Asp Ala Val Glu Asn Cys
                        30                  35                  40
   ggc tcc ggc cac cca ggc acc gca atg agc ctg gct ccc ctt gca tac   375
   Gly Ser Gly His Pro Gly Thr Ala Met Ser Leu Ala Pro Leu Ala Tyr
                    45                  50                  55
   acc ttg tac cag cgg gtt atg aac gta gat cca cag gac acc aac tgg   423
   Thr Leu Tyr Gln Arg Val Met Asn Val Asp Pro Gln Asp Thr Asn Trp
            60                  65                  70
   gca ggc cgt gac cgc ttc gtt ctt tct tgt ggc cac tcc tct ttg acc   471
   Ala Gly Arg Asp Arg Phe Val Leu Ser Cys Gly His Ser Ser Leu Thr
        75                  80                  85
   cag tac atc cag ctt tac ttg ggt gga ttc ggc ctt gag atg gat gac   519
   Gln Tyr Ile Gln Leu Tyr Leu Gly Gly Phe Gly Leu Glu Met Asp Asp
    90                  95                 100                 105
   ctg aag gct ctg cgc acc tgg gat tcc ttg acc cca gga cac cct gag   567
   Leu Lys Ala Leu Arg Thr Trp Asp Ser Leu Thr Pro Gly His Pro Glu
               110                 115                 120
   tac cgc cac acc aag ggc gtt gag atc acc act ggc cct ctt ggc cag    615
   Tyr Arg His Thr Lys Gly Val Glu Ile Thr Thr Gly Pro Leu Gly Gln
           125                 130                 135
   ggt ctt gca tct gca gtt ggt atg gcc atg gct gct cgt cgt gag cgt    663
   Gly Leu Ala Ser Ala Val Gly Met Ala Met Ala Ala Arg Arg Glu Arg
       140                 145                 150
   ggc cta ttc gac cca acc gct gct gag ggc gaa tcc cca ttc gac cac    711
   Gly Leu Phe Asp Pro Thr Ala Ala Glu Gly Glu Ser Pro Phe Asp His
   155                 160                 165
   cac atc tac gtc att gct tct gat ggt gac ctg cag gaa ggt gtc acc    759
   His Ile Tyr Val Ile Ala Ser Asp Gly Asp Leu Gln Glu Gly Val Thr
   170                 175                 180                 185
   tct gag gca tcc tcc atc gct ggc acc cag cag ctg ggc aac ctc atc    807
   Ser Glu Ala Ser Ser Ile Ala Gly Thr Gln Gln Leu Gly Asn Leu Ile
                   190                 195                 200
   gtg ttc tgg gat gac aac cgc atc tcc atc gaa gac aac act gag atc    855
   Val Phe Trp Asp Asp Asn Arg Ile Ser Ile Glu Asp Asn Thr Glu Ile
               205                 210                 215
   gct ttc aac gag gac gtt gtt gct cgt tac aag gct tac ggc tgg cag    903
   Ala Phe Asn Glu Asp Val Val Ala Arg Tyr Lys Ala Tyr Gly Trp Gln
           220                 225                 230
   acc att gag gtt gag gct ggc gag gac gtt gca gca atc gaa gct gca    951
   Thr Ile Glu Val Glu Ala Gly Glu Asp Val Ala Ala Ile Glu Ala Ala
       235                 240                 245
   gtg gct gag gct aag aag gac acc aag cga cct acc ttc atc cgc gtt    999
   Val Ala Glu Ala Lys Lys Asp Thr Lys Arg Pro Thr Phe Ile Arg Val
   250                 255                 260                 265
   cgc acc atc atc ggc ttc cca gct cca act atg atg aac acc ggt gct    1047
   Arg Thr Ile Ile Gly Phe Pro Ala Pro Thr Met Met Asn Thr Gly Ala
                   270                 275                 280
   gtg cac ggt gct gct ctt ggc gca cgt gaa gtt gca gca acc aag act    1095
   Val His Gly Ala Ala Leu Gly Ala Arg Glu Val Ala Ala Thr Lys Thr
               285                 290                 295
   gag ctt gga ttc gat cct gag gct cac ttc gcg atc gac gat gag gtt    1143
   Glu Leu Gly Phe Asp Pro Glu Ala His Phe Ala Ile Asp Asp Glu Val
           300                 305                 310
   atc gct cac acc cgc tcc ctc gca gag cgc gct gca cag aag aag gct    1191
   Ile Ala His Thr Arg Ser Leu Ala Glu Arg Ala Ala Gln Lys Lys Ala
       315                 320                 325
   gca tgg cag gtc aag ttc gat gag tgg gca gct gcc aac cct gag aac    1239
   Ala Trp Gln Val Lys Phe Asp Glu Trp Ala Ala Ala Asn Pro Glu Asn
   330                 335                 340                 345
   aag gct ctg ttc gat cgc ctg aac tcc cgt gag ctt cca gcg ggc tac    1287
   Lys Ala Leu Phe Asp Arg Leu Asn Ser Arg Glu Leu Pro Ala Gly Tyr
                   350                 355                 360
   gct gac gag ctc cca aca tgg gat gca gat gag aag ggc gtc gca act    1335
   Ala Asp Glu Leu Pro Thr Trp Asp Ala Asp Glu Lys Gly Val Ala Thr
               365                 370                 375
   cgt aag gct tcc gag gct gca ctt cag gca ctg ggc aag acc ctt cct     1383
   Arg Lys Ala Ser Glu Ala Ala Leu Gln Ala Leu Gly Lys Thr Leu Pro
           380                 385                 390
   gag ctg tgg ggc ggt tcc gct gac ctc gca ggt tcc aac aac acc gtg     1431
   Glu Leu Trp Gly Gly Ser Ala Asp Leu Ala Gly Ser Asn Asn Thr Val
       395                 400                 405
   atc aag ggc tcc cct tcc ttc ggc cct gag tcc atc tcc acc gag acc     1479
   Ile Lys Gly Ser Pro Ser Phe Gly Pro Glu Ser Ile Ser Thr Glu Thr
   410                 415                 420                 425
   tgg tct gct gag cct tac ggc cgt aac ctg cac ttc ggt atc cgt gag     1527
   Trp Ser Ala Glu Pro Tyr Gly Arg Asn Leu His Phe Gly Ile Arg Glu
                   430                 435                 440
   cac gct atg gga tcc atc ctc aac ggc att tcc ctc cac ggt ggc acc     1575
   His Ala Met Gly Ser Ile Leu Asn Gly Ile Ser Leu His Gly Gly Thr
               445                 450                 455
   cgc cca tac ggc gga acc ttc ctc atc ttc tcc gac tac atg cgt cct     1623
   Arg Pro Tyr Gly Gly Thr Phe Leu Ile Phe Ser Asp Tyr Met Arg Pro
           460                 465                 470
   gca gtt cgt ctt gca gct ctc atg gag acc gac gct tac tac gtc tgg     1671
   Ala Val Arg Leu Ala Ala Leu Met Glu Thr Asp Ala Tyr Tyr Val Trp
       475                 480                 485
   acc cac gac tcc atc ggt ctg ggc gaa gat ggc cca acc cac cag cct     1719
   Thr His Asp Ser Ile Gly Leu Gly Glu Asp Gly Pro Thr His Gln Pro
   490                 495                 500                 505
   gtt gaa acc ttg gct gca ctg cgc gcc atc cca ggt ctg tcc gtc ctg     1767
   Val Glu Thr Leu Ala Ala Leu Arg Ala Ile Pro Gly Leu Ser Val Leu
                   510                 515                 520
   cgt cct gca gat gcg aac gag acc gcc cag gct tgg gct gca gca ctt     1815
   Arg Pro Ala Asp Ala Asn Glu Thr Ala Gln Ala Trp Ala Ala Ala Leu
               525                 530                 535
   gag tac aag gaa ggc cct aag ggt ctt gca ctg acc cgc cag aac gtt     1863
   Glu Tyr Lys Glu Gly Pro Lys Gly Leu Ala Leu Thr Arg Gln Asn Val
           540                 545                 550
   cct gtt ctg gaa ggc acc aag gag aag gct gct gaa ggc gtt cgc cgc     1911
   Pro Val Leu Glu Gly Thr Lys Glu Lys Ala Ala Glu Gly Val Arg Arg
       555                 560                 565
   ggt ggc tac gtc ctg gtt gag ggt tcc aag gaa acc cca gat gtg atc     1959
   Gly Gly Tyr Val Leu Val Glu Gly Ser Lys Glu Thr Pro Asp Val Ile
   570                 575                 580                 585
   ctc atg ggc tcc ggc tcc gag gtt cag ctt gca gtt aac gct gcg aag     2007
   Leu Met Gly Ser Gly Ser Glu Val Gln Leu Ala Val Asn Ala Ala Lys
                   590                 595                 600
   gct ctg gaa gct gag ggc gtt gca gct cgc gtt gtt tcc gtt cct tgc     2055
   Ala Leu Glu Ala Glu Gly Val Ala Ala Arg Val Val Ser Val Pro Cys
               605                 610                 615
   atg gat tgg ttc cag gag cag gac gca gag tac atc gag tcc gtt ctg     2103
   Met Asp Trp Phe Gln Glu Gln Asp Ala Glu Tyr Ile Glu Ser Val Leu
           620                 625                 630
   cct gca gct gtg acc gct cgt gtg tct gtt gaa gct ggc atc gca atg    2151
   Pro Ala Ala Val Thr Ala Arg Val Ser Val Glu Ala Gly Ile Ala Met
       635                 640                 645
   cct tgg tac cgc ttc ttg ggc acc cag ggc cgt gct gtc tcc ctt gag    2199
   Pro Trp Tyr Arg Phe Leu Gly Thr Gln Gly Arg Ala Val Ser Leu Glu
   650                 655                 660                 665
   cac ttc ggt gct tct gcg gat tac cag acc ctg ttt gag aag ttc ggc    2247
   His Phe Gly Ala Ser Ala Asp Tyr Gln Thr Leu Phe Glu Lys Phe Gly
                   670                 675                 680
   atc acc acc gat gca gtc gtg gca gcg gcc aag gac tcc att aac ggt    2295
   Ile Thr Thr Asp Ala Val Val Ala Ala Ala Lys Asp Ser Ile Asn Gly
               685                 690                 695
   taattgccct gctgttttta gcttcaaccc ggggcaatat gattctccgg aattt       2350
<210> 2
   <211> 697
   <212> PRT
   <213> Corynebacterium glutamicum
<400> 2
   Leu Thr Leu Ser Pro Glu Leu Gln Ala Leu Thr Val Arg Asn Tyr Pro
   1               5                   10                  15
   Ser Asp Trp Ser Asp Val Asp Thr Lys Ala Val Asp Thr Val Arg Val
               20                  25                  30
   Leu Ala Ala Asp Ala Val Glu Asn Cys Gly Ser Gly His Pro Gly Thr
           35                  40                  45
   Ala Met Ser Leu Ala Pro Leu Ala Tyr Thr Leu Tyr Gln Arg Val Met
       50                  55                  60
   Asn Val Asp Pro Gln Asp Thr Asn Trp Ala Gly Arg Asp Arg Phe Val
   65                  70                  75                  80
   Leu Ser Cys Gly His Ser Ser Leu Thr Gln Tyr Ile Gln Leu Tyr Leu
                   85                  90                  95
   Gly Gly Phe Gly Leu Glu Met Asp Asp Leu Lys Ala Leu Arg Thr Trp
               100                 105                 110
   Asp Ser Leu Thr Pro Gly His Pro Glu Tyr Arg His Thr Lys Gly Val
           115                 120                 125
   Glu Ile Thr Thr Gly Pro Leu Gly Gln Gly Leu Ala Ser Ala Val Gly
       130                 135                 140
   Met Ala Met Ala Ala Arg Arg Glu Arg Gly Leu Phe Asp Pro Thr Ala
   145                 150                 155                 160
   Ala Glu Gly Glu Ser Pro Phe Asp His His Ile Tyr Val Ile Ala Ser
                   165                 170                 175
   Asp Gly Asp Leu Gln Glu Gly Val Thr Ser Glu Ala Ser Ser Ile Ala
               180                 185                 190
   Gly Thr Gln Gln Leu Gly Asn Leu Ile Val Phe Trp Asp Asp Asn Arg
           195                 200                 205
   Ile Ser Ile Glu Asp Asn Thr Glu Ile Ala Phe Asn Glu Asp Val Val
       210                 215                 220
   Ala Arg Tyr Lys Ala Tyr Gly Trp Gln Thr Ile Glu Val Glu Ala Gly
   225                 230                 235                 240
   Glu Asp Val Ala Ala Ile Glu Ala Ala Val Ala Glu Ala Lys Lys Asp
                   245                 250                 255
   Thr Lys Arg Pro Thr Phe Ile Arg Val Arg Thr Ile Ile Gly Phe Pro
               260                 265                 270
   Ala Pro Thr Met Met Asn Thr Gly Ala Val His Gly Ala Ala Leu Gly
           275                 280                 285
   Ala Arg Glu Val Ala Ala Thr Lys Thr Glu Leu Gly Phe Asp Pro Glu
       290                 295                 300
   Ala His Phe Ala Ile Asp Asp Glu Val Ile Ala His Thr Arg Ser Leu
   305                 310                 315                 320
   Ala Glu Arg Ala Ala Gln Lys Lys Ala Ala Trp Gln Val Lys Phe Asp
                   325                 330                 335
   Glu Trp Ala Ala Ala Asn Pro Glu Asn Lys Ala Leu Phe Asp Arg Leu
               340                 345                 350
   Asn Ser Arg Glu Leu Pro Ala Gly Tyr Ala Asp Glu Leu Pro Thr Trp
           355                 360                 365
   Asp Ala Asp Glu Lys Gly Val Ala Thr Arg Lys Ala Ser Glu Ala Ala
       370                 375                 380
   Leu Gln Ala Leu Gly Lys Thr Leu Pro Glu Leu Trp Gly Gly Ser Ala
   385                 390                 395                 400
   Asp Leu Ala Gly Ser Asn Asn Thr Val Ile Lys Gly Ser Pro Ser Phe
                   405                 410                 415
   Gly Pro Glu Ser Ile Ser Thr Glu Thr Trp Ser Ala Glu Pro Tyr Gly
               420                 425                 430
   Arg Asn Leu His Phe Gly Ile Arg Glu His Ala Met Gly Ser Ile Leu
           435                 440                 445
   Asn Gly Ile Ser Leu His Gly Gly Thr Arg Pro Tyr Gly Gly Thr Phe
       450                 455                 460
   Leu Ile Phe Ser Asp Tyr Met Arg Pro Ala Val Arg Leu Ala Ala Leu
   465                 470                 475                 480
   Met Glu Thr Asp Ala Tyr Tyr Val Trp Thr His Asp Ser Ile Gly Leu
                   485                 490                 495
   Gly Glu Asp Gly Pro Thr His Gln Pro Val Glu Thr Leu Ala Ala Leu
               500                 505                 510
   Arg Ala Ile Pro Gly Leu Ser Val Leu Arg Pro Ala Asp Ala Asn Glu
           515                 520                 525
   Thr Ala Gln Ala Trp Ala Ala Ala Leu Glu Tyr Lys Glu Gly Pro Lys
       530                 535                 540
   Gly Leu Ala Leu Thr Arg Gln Asn Val Pro Val Leu Glu Gly Thr Lys
   545 550 555 560
   Glu Lys Ala Ala Glu Gly Val Arg Arg Gly Gly Tyr Val Leu Val Glu
   565 570 575
   Gly Ser Lys Glu Thr Pro Asp Val Ile Leu Met Gly Ser Gly Ser Glu
   580 585 590
   Val Gln Leu Ala Val Asn Ala Ala Lys Ala Leu Glu Ala Glu Gly Val
   595 600 605
   Ala Ala Arg Val Val Ser Val Pro Cys Met Asp Trp Phe Gln Glu Gln
   610 615 620
   Asp Ala Glu Tyr Ile Glu Ser Val Leu Pro Ala Ala Val Thr Ala Arg
   625 630 635 640
   Val Ser Val Glu Ala Gly Ile Ala Met Pro Trp Tyr Arg Phe Leu Gly
   645 650 655
   Thr Gln Gly Arg Ala Val Ser Leu Glu His Phe Gly Ala Ser Ala Asp
   660 665 670
   Tyr Gln Thr Leu Phe Glu Lys Phe Gly Ile Thr Thr Asp Ala Val Val
   675 680 685
   Ala Ala Ala Lys Asp Ser Ile Asn Gly
   690 695
<210> 3
   <211> 2160
   <212> DNA
   <213> Corynebacterium glutamicum
<220>
   <221> CDS
   <222> (327)..(2063)
<400> 3
   ttagaggcga ttctgtgagg tcactttttg tggggtcggg gtctaaattt ggccagtttt 60
   cgaggcgacc agacaggcgt gcccacgatg tttaaatagg cgatcggtgg gcatctgtgt 120
   ttggtttcga cgggctgaaa ccaaaccaga ctgcccagca acgacggaaa tcccaaaagt 180
   gggcatccct gtttggtacc gagtacccac ccgggcctga aactccctgg caggcgggcg 240
   aagcgtggca acaactggaa tttaagagca caattgaagt cgcaccaagt taggcaacac 300
   aatagccata acgttgagga gttcag atg gca cac agc tac gca gaa caa tta  353
                                Met Ala His Ser Tyr Ala Glu Gln Leu
                                 1               5
   att gac act ttg gaa gct caa ggt gtg aag cga att tat ggt ttg gtg   401
   Ile Asp Thr Leu Glu Ala Gln Gly Val Lys Arg Ile Tyr Gly Leu Val
    10                  15                  20                  25
   ggt gac agc ctt aat ccg atc gtg gat gct gtc cgc caa tca gat att   449
   Gly Asp Ser Leu Asn Pro Ile Val Asp Ala Val Arg Gln Ser Asp Ile
                    30                  35                  40
   gag tgg gtg cac gtt cga aat gag gaa gcg gcg gcg ttt gca gcc ggt    497
   Glu Trp Val His Val Arg Asn Glu Glu Ala Ala Ala Phe Ala Ala Gly
               45                  50                  55
   gcg gaa tcg ttg atc act ggg gag ctg gca gta tgt gct gct tct tgt    545
   Ala Glu Ser Leu Ile Thr Gly Glu Leu Ala Val Cys Ala Ala Ser Cys
           60                  65                  70
   ggt cct gga aac aca cac ctg att cag ggt ctt tat gat tcg cat cga    593
   Gly Pro Gly Asn Thr His Leu Ile Gln Gly Leu Tyr Asp Ser His Arg
       75                  80                  85
   aat ggt gcg aag gtg ttg gcc atc gct agc cat att ccg agt gcc cag    641
   Asn Gly Ala Lys Val Leu Ala Ile Ala Ser His Ile Pro Ser Ala Gln
   90                  95                  100                 105
   att ggt tcg acg ttc ttc cag gaa acg cat ccg gag att ttg ttt aag    689
   Ile Gly Ser Thr Phe Phe Gln Glu Thr His Pro Glu Ile Leu Phe Lys
                   110                 115                 120
   gaa tgc tct ggt tac tgc gag atg gtg aat ggt ggt gag cag ggt gaa    737
   Glu Cys Ser Gly Tyr Cys Glu Met Val Asn Gly Gly Glu Gln Gly Glu
               125                 130                 135
   cgc att ttg cat cac gcg att cag tcc acc atg gcg ggt aaa ggt gtg    785
   Arg Ile Leu His His Ala Ile Gln Ser Thr Met Ala Gly Lys Gly Val
           140                 145                 150
   tcg gtg gta gtg att cct ggt gat atc gct aag gaa gac gca ggt gac    833
   Ser Val Val Val Ile Pro Gly Asp Ile Ala Lys Glu Asp Ala Gly Asp
       155                 160                 165
   ggt act tat tcc aat tcc act att tct tct ggc act cct gtg gtg ttc    881
   Gly Thr Tyr Ser Asn Ser Thr Ile Ser Ser Gly Thr Pro Val Val Phe
   170                 175                 180                 185
   ccg gat cct act gag gct gca gcg ctg gtg gag gcg att aac aac gct    929
   Pro Asp Pro Thr Glu Ala Ala Ala Leu Val Glu Ala Ile Asn Asn Ala
                   190                 195                 200
   aag tct gtc act ttg ttc tgc ggt gcg ggc gtg aag aat gct cgc gcg    977
   Lys Ser Val Thr Leu Phe Cys Gly Ala Gly Val Lys Asn Ala Arg Ala
               205                 210                 215
   cag gtg ttg gag ttg gcg gag aag att aaa tca ccg atc ggg cat gcg    1025
   Gln Val Leu Glu Leu Ala Glu Lys Ile Lys Ser Pro Ile Gly His Ala
           220                 225                 230
   ctg ggt ggt aag cag tac atc cag cat gag aat ccg ttt gag gtc ggc    1073
   Leu Gly Gly Lys Gln Tyr Ile Gln His Glu Asn Pro Phe Glu Val Gly
       235                 240                 245
   atg tct ggc ctg ctt ggt tac ggc gcc tgc gtg gat gcg tcc aat gag    1121
   Met Ser Gly Leu Leu Gly Tyr Gly Ala Cys Val Asp Ala Ser Asn Glu
   250                 255                 260                 265
   gcg gat ctg ctg att cta ttg ggt acg gat ttc cct tat tct gat ttc    1169
   Ala Asp Leu Leu Ile Leu Leu Gly Thr Asp Phe Pro Tyr Ser Asp Phe
                   270                 275                 280
   ctt cct aaa gac aac gtt gcc cag gtg gat atc aac ggt gcg cac att    1217
   Leu Pro Lys Asp Asn Val Ala Gln Val Asp Ile Asn Gly Ala His Ile
               285                 290                 295
   ggt cga cgt acc acg gtg aag tat ccg gtg acc ggt gat gtt gct gca   1265
   Gly Arg Arg Thr Thr Val Lys Tyr Pro Val Thr Gly Asp Val Ala Ala
           300                 305                 310
   aca atc gaa aat att ttg cct cat gtg aag gaa aaa aca gat cgt tcc   1313
   Thr Ile Glu Asn Ile Leu Pro His Val Lys Glu Lys Thr Asp Arg Ser
       315                 320                 325
   ttc ctt gat cgg atg ctc aag gca cac gag cgt aag ttg agc tcg gtg   1361
   Phe Leu Asp Arg Met Leu Lys Ala His Glu Arg Lys Leu Ser Ser Val
   330                 335                 340                 345
   gta gag acg tac aca cat aac gtc gag aag cat gtg cct att cac cct   1409
   Val Glu Thr Tyr Thr His Asn Val Glu Lys His Val Pro Ile His Pro
                   350                 355                 360
   gaa tac gtt gcc tct att ttg aac gag ctg gcg gat aag gat gcg gtg   1457
   Glu Tyr Val Ala Ser Ile Leu Asn Glu Leu Ala Asp Lys Asp Ala Val
               365                 370                 375
   ttt act gtg gat acc ggc atg tgc aat gtg tgg cat gcg agg tac atc   1505
   Phe Thr Val Asp Thr Gly Met Cys Asn Val Trp His Ala Arg Tyr Ile
           380                 385                 390
   gag aat ccg gag gga acg cgc gac ttt gtg ggt tca ttc cgc cac ggc   1553
   Glu Asn Pro Glu Gly Thr Arg Asp Phe Val Gly Ser Phe Arg His Gly
       395                 400                 405
   acg atg gct aat gcg ttg cct cat gcg att ggt gcg caa agt gtt gat   1601
   Thr Met Ala Asn Ala Leu Pro His Ala Ile Gly Ala Gln Ser Val Asp
   410                 415                 420                 425
   cga aac cgc cag gtg atc gcg atg tgt ggc gat ggt ggt ttg ggc atg   1649
   Arg Asn Arg Gln Val Ile Ala Met Cys Gly Asp Gly Gly Leu Gly Met
                   430                 435                 440
   ctg ctg ggt gag ctt ctg acc gtt aag ctg cac caa ctt ccg ctg aag   1697
   Leu Leu Gly Glu Leu Leu Thr Val Lys Leu His Gln Leu Pro Leu Lys
               445                 450                 455
   gct gtg gtg ttt aac aac agt tct ttg ggc atg gtg aag ttg gag atg   1745
   Ala Val Val Phe Asn Asn Ser Ser Leu Gly Met Val Lys Leu Glu Met
           460                 465                 470
   ctc gtg gag gga cag cca gaa ttt ggt act gac cat gag gaa gtg aat   1793
   Leu Val Glu Gly Gln Pro Glu Phe Gly Thr Asp His Glu Glu Val Asn
       475                 480                 485
   ttc gca gag att gcg gcg gct gcg ggt atc aaa tcg gta cgc atc acc   1841
   Phe Ala Glu Ile Ala Ala Ala Ala Gly Ile Lys Ser Val Arg Ile Thr
   490                 495                 500                 505
   gat ccg aag aaa gtt cgc gag cag cta gct gag gca ttg gca tat cct   1889
   Asp Pro Lys Lys Val Arg Glu Gln Leu Ala Glu Ala Leu Ala Tyr Pro
                   510                 515                 520
   gga cct gta ctg atc gat atc gtc acg gat cct aat gcg ctg tcg atc   1937
   Gly Pro Val Leu Ile Asp Ile Val Thr Asp Pro Asn Ala Leu Ser Ile
               525                 530                 535
   cca cca acc atc acg tgg gaa cag gtc atg gga ttc agc aag gcg gcc   1985
   Pro Pro Thr Ile Thr Trp Glu Gln Val Met Gly Phe Ser Lys Ala Ala
           540                 545                 550
   acc cga acc gtc ttt ggt gga gga gta gga gcg atg atc gat ctg gcc   2033
   Thr Arg Thr Val Phe Gly Gly Gly Val Gly Ala Met Ile Asp Leu Ala
       555                 560                 565
   cgt tcg aac ata agg aat att cct act cca tgatgattga tacacctgct     2083
   Arg Ser Asn Ile Arg Asn Ile Pro Thr Pro
   570                 575
   gttctcattg accgcgagcg cttaactgcc aacatttcca ggatggcagc tcacgccggt 2143
   gcccatgaga ttgccct                                                 2160
<210> 4
   <211> 579
   <212> PRT
   <213> Corynebacterium glutamicum
<400> 4
   Met Ala His Ser Tyr Ala Glu Gln Leu Ile Asp Thr Leu Glu Ala Gln
   1               5                   10                  15
   Gly Val Lys Arg Ile Tyr Gly Leu Val Gly Asp Ser Leu Asn Pro Ile
               20                  25                  30
   Val Asp Ala Val Arg Gln Ser Asp Ile Glu Trp Val His Val Arg Asn
           35                  40                  45
   Glu Glu Ala Ala Ala Phe Ala Ala Gly Ala Glu Ser Leu Ile Thr Gly
       50                  55                  60
   Glu Leu Ala Val Cys Ala Ala Ser Cys Gly Pro Gly Asn Thr His Leu
   65                  70                  75                  80
   Ile Gln Gly Leu Tyr Asp Ser His Arg Asn Gly Ala Lys Val Leu Ala
                   85                  90                  95
   Ile Ala Ser His Ile Pro Ser Ala Gln Ile Gly Ser Thr Phe Phe Gln
               100                 105                 110
   Glu Thr His Pro Glu Ile Leu Phe Lys Glu Cys Ser Gly Tyr Cys Glu
           115                 120                 125
   Met Val Asn Gly Gly Glu Gln Gly Glu Arg Ile Leu His His Ala Ile
       130                 135                 140
   Gln Ser Thr Met Ala Gly Lys Gly Val Ser Val Val Val Ile Pro Gly
   145                 150                 155                 160
   Asp Ile Ala Lys Glu Asp Ala Gly Asp Gly Thr Tyr Ser Asn Ser Thr
                   165                 170                 175
   Ile Ser Ser Gly Thr Pro Val Val Phe Pro Asp Pro Thr Glu Ala Ala
               180                 185                 190
   Ala Leu Val Glu Ala Ile Asn Asn Ala Lys Ser Val Thr Leu Phe Cys
           195                 200                 205
   Gly Ala Gly Val Lys Asn Ala Arg Ala Gln Val Leu Glu Leu Ala Glu
       210                 215                 220
   Lys Ile Lys Ser Pro Ile Gly His Ala Leu Gly Gly Lys Gln Tyr Ile
   225                 230                 235                 240
   Gln His Glu Asn Pro Phe Glu Val Gly Met Ser Gly Leu Leu Gly Tyr
   245 250 255
   Gly Ala Cys Val Asp Ala Ser Asn Glu Ala Asp Leu Leu Ile Leu Leu
   260 265 270
   Gly Thr Asp Phe Pro Tyr Ser Asp Phe Leu Pro Lys Asp Asn Val Ala
   275 280 285
   Gln Val Asp Ile Asn Gly Ala His Ile Gly Arg Arg Thr Thr Val Lys
   290 295 300
   Tyr Pro Val Thr Gly Asp Val Ala Ala Thr Ile Glu Asn Ile Leu Pro
   305 310 315 320
   His Val Lys Glu Lys Thr Asp Arg Ser Phe Leu Asp Arg Met Leu Lys
   325 330 335
   Ala His Glu Arg Lys Leu Ser Ser Val Val Glu Thr Tyr Thr His Asn
   340 345 350
   Val Glu Lys His Val Pro Ile His Pro Glu Tyr Val Ala Ser Ile Leu
   355 360 365
   Asn Glu Leu Ala Asp Lys Asp Ala Val Phe Thr Val Asp Thr Gly Met
   370 375 380
   Cys Asn Val Trp His Ala Arg Tyr Ile Glu Asn Pro Glu Gly Thr Arg
   385 390 395 400
   Asp Phe Val Gly Ser Phe Arg His Gly Thr Met Ala Asn Ala Leu Pro
   405 410 415
   His Ala Ile Gly Ala Gln Ser Val Asp Arg Asn Arg Gln Val Ile Ala
   420 425 430
   Met Cys Gly Asp Gly Gly Leu Gly Met Leu Leu Gly Glu Leu Leu Thr
   435 440 445
   Val Lys Leu His Gln Leu Pro Leu Lys Ala Val Val Phe Asn Asn Ser
   450 455 460
   Ser Leu Gly Met Val Lys Leu Glu Met Leu Val Glu Gly Gln Pro Glu
   465 470 475 480
   Phe Gly Thr Asp His Glu Glu Val Asn Phe Ala Glu Ile Ala Ala Ala
   485 490 495
   Ala Gly Ile Lys Ser Val Arg Ile Thr Asp Pro Lys Lys Val Arg Glu
   500 505 510
   Gln Leu Ala Glu Ala Leu Ala Tyr Pro Gly Pro Val Leu Ile Asp Ile
   515 520 525
   Val Thr Asp Pro Asn Ala Leu Ser Ile Pro Pro Thr Ile Thr Trp Glu
   530 535 540
   Gln Val Met Gly Phe Ser Lys Ala Ala Thr Arg Thr Val Phe Gly Gly
   545 550 555 560
   Gly Val Gly Ala Met Ile Asp Leu Ala Arg Ser Asn Ile Arg Asn Ile
   565 570 575
   Pro Thr Pro
<210> 5
   <211> 875
   <212> DNA
   <213> Corynebacterium glutamicum
<400> 5

### SEQUENCE PROTOCOL

<110> National University of Ireland, Galway Degussa-Hüls AG
<120> Process for the fermentative preparation of L-amino acids using coryneform bacteria.
<130> 000019 BT
<140>
   <141>
<160> 5
<170> Patent In Ver. 2.1
<210> 1
   <211> 2350
   <212> DNA
   <213> Corynebacterium glutamicum
<220>
   <221> CDS
   <222> (205)..(2295)
   <223> tkt
<400> 1
   cgggtcagat taagcaaaga ctactttcgg ggtagatcac ctttgccaaa tttgaaccaa 60
   ttaacctaag tcgtagatct gatcatcgga tctaacgaaa acgaaccaaa actttggtcc 120
   cggtttaacc caggaaggat tgaccaccta acgaaaacga accaaaactt tggtcccggt 180
   ttaacccagg aaggattgac cacc ttg acg ctg tca cct gaa ctt cag gcg   231
                              Leu Thr Leu Ser Pro Glu Leu Gln Ala
                              1               5
   ctc act gta cgc aat tac ccc tct gat tgg tcc gat gtg gac acc aag   279
   Leu Thr Val Arg Asn Tyr Pro Ser Asp Trp Ser Asp Val Asp Thr Lys
    10                 15                  20                  25
   gct gta gac act gtt cgt gtc ctc gct gca gac gct gta gaa aac tgt   327
   Ala Val Asp Thr Val Arg Val Leu Ala Ala Asp Ala Val Glu Asn Cys
                   30                  35                  40
   ggc tcc ggc cac cca ggc acc gca atg agc ctg gct ccc ctt gca tac   375
   Gly Ser Gly His Pro Gly Thr Ala Met Ser Leu Ala Pro Leu Ala Tyr
               45                  50                  55
   acc ttg tac cag cgg gtt atg aac gta gat cca cag gac acc aac tgg   423
   Thr Leu Tyr Gln Arg Val Met Asn Val Asp Pro Gln Asp Thr Asn Trp
           60                  65                  70
   gca ggc cgt gac cgc ttc gtt ctt tct tgt ggc cac tcc tct ttg acc   471
   Ala Gly Arg Asp Arg Phe Val Leu Ser Cys Gly His Ser Ser Leu Thr
       75                  80                  85
   cag tac atc cag ctt tac ttg ggt gga ttc ggc ctt gag atg gat gac   519
   Gln Tyr Ile Gln Leu Tyr Leu Gly Gly Phe Gly Leu Glu Met Asp Asp
    90                 95                 100                 105
   ctg aag gct ctg cgc acc tgg gat tcc ttg acc cca gga cac cct gag   567
   Leu Lys Ala Leu Arg Thr Trp Asp Ser Leu Thr Pro Gly His Pro Glu
                       110                 115                 120
   tac cgc cac acc aag ggc gtt gag atc acc act ggc cct ctt ggc cag      615
   Tyr Arg His Thr Lys Gly Val Glu Ile Thr Thr Gly Pro Leu Gly Gln
                   125                 130                 135
   ggt ctt gca tct gca gtt ggt atg gcc atg gct gct cgt cgt gag cgt      663
   Gly Leu Ala Ser Ala Val Gly Met Ala Met Ala Ala Arg Arg Glu Arg
               140                 145                 150
   ggc cta ttc gac cca acc gct gct gag ggc gaa tcc cca ttc gac cac      711
   Gly Leu Phe Asp Pro Thr Ala Ala Glu Gly Glu Ser Pro Phe Asp His
       155                 160                 165
   cac atc tac gtc att gct tct gat ggt gac ctg cag gaa ggt gtc acc      759
   His Ile Tyr Val Ile Ala Ser Asp Gly Asp Leu Gln Glu Gly Val Thr
   170                 175                 180                 185
   tct gag gca tcc tcc atc gct ggc acc cag cag ctg ggc aac ctc atc      807
   Ser Glu Ala Ser Ser Ile Ala Gly Thr Gln Gln Leu Gly Asn Leu Ile
                   190                 195                 200
   gtg ttc tgg gat gac aac cgc atc tcc atc gaa gac aac act gag atc      855
   Val Phe Trp Asp Asp Asn Arg Ile Ser Ile Glu Asp Asn Thr Glu Ile
               205                 210                 215
   gct ttc aac gag gac gtt gtt gct cgt tac aag gct tac ggc tgg cag      903
   Ala Phe Asn Glu Asp Val Val Ala Arg Tyr Lys Ala Tyr Gly Trp Gln
           220                 225                 230
   acc att gag gtt gag gct ggc gag gac gtt gca gca atc gaa gct gca      951
   Thr Ile Glu Val Glu Ala Gly Glu Asp Val Ala Ala Ile Glu Ala Ala
       235                 240                 245
   gtg gct gag gct aag aag gac acc aag cga cct acc ttc atc cgc gtt      999
   Val Ala Glu Ala Lys Lys Asp Thr Lys Arg Pro Thr Phe Ile Arg Val
   250                 255                 260                 265
   cgc acc atc atc ggc ttc cca gct cca act atg atg aac acc ggt gct      1047
   Arg Thr Ile Ile Gly Phe Pro Ala Pro Thr Met Met Asn Thr Gly Ala
                   270                 275                 280
   gtg cac ggt gct gct ctt ggc gca cgt gaa gtt gca gca acc aag act      1095
   Val His Gly Ala Ala Leu Gly Ala Arg Glu Val Ala Ala Thr Lys Thr
               285                 290                 295
   gag ctt gga ttc gat cct gag gct cac ttc gcg atc gac gat gag gtt      1143
   Glu Leu Gly Phe Asp Pro Glu Ala His Phe Ala Ile Asp Asp Glu Val
           300                 305                 310
   atc gct cac acc cgc tcc ctc gca gag cgc gct gca cag aag aag gct      1191
   Ile Ala His Thr Arg Ser Leu Ala Glu Arg Ala Ala Gln Lys Lys Ala
       315                 320                 325
   gca tgg cag gtc aag ttc gat gag tgg gca gct gcc aac cct gag aac      1239
   Ala Trp Gln Val Lys Phe Asp Glu Trp Ala Ala Ala Asn Pro Glu Asn
   330                 335                 340                 345
   aag gct ctg ttc gat cgc ctg aac tcc cgt gag ctt cca gcg ggc tac      1287
   Lys Ala Leu Phe Asp Arg Leu Asn Ser Arg Glu Leu Pro Ala Gly Tyr
                   350                 355                 360
   gct gac gag ctc cca aca tgg gat gca gat gag aag ggc gtc gca act      1335
   Ala Asp Glu Leu Pro Thr Trp Asp Ala Asp Glu Lys Gly Val Ala Thr
           365                 370                 375
   cgt aag gct tcc gag gct gca ctt cag gca ctg ggc aag acc ctt cct    1383
   Arg Lys Ala Ser Glu Ala Ala Leu Gln Ala Leu Gly Lys Thr Leu Pro
               380                 385                 390
   gag ctg tgg ggc ggt tcc gct gac ctc gca ggt tcc aac aac acc gtg    1431
   Glu Leu Trp Gly Gly Ser Ala Asp Leu Ala Gly Ser Asn Asn Thr Val
       395                 400                 405
   atc aag ggc tcc cct tcc ttc ggc cct gag tcc atc tcc acc gag acc    1479
   Ile Lys Gly Ser Pro Ser Phe Gly Pro Glu Ser Ile Ser Thr Glu Thr
   410                 415                 420                 425
   tgg tct gct gag cct tac ggc cgt aac ctg cac ttc ggt atc cgt gag    1527
   Trp Ser Ala Glu Pro Tyr Gly Arg Asn Leu His Phe Gly Ile Arg Glu
                   430                 435                 440
   cac gct atg gga tcc atc ctc aac ggc att tcc ctc cac ggt ggc acc    1575
   His Ala Met Gly Ser Ile Leu Asn Gly Ile Ser Leu His Gly Gly Thr
               445                 450                 455
   cgc cca tac ggc gga acc ttc ctc atc ttc tcc gac tac atg cgt cct    1623
   Arg Pro Tyr Gly Gly Thr Phe Leu Ile Phe Ser Asp Tyr Met Arg Pro
           460                 465                 470
   gca gtt cgt ctt gca gct ctc atg gag acc gac gct tac tac gtc tgg    1671
   Ala Val Arg Leu Ala Ala Leu Met Glu Thr Asp Ala Tyr Tyr Val Trp
       475                 480                 485
   acc cac gac tcc atc ggt ctg ggc gaa gat ggc cca acc cac cag cct    1719
   Thr His Asp Ser Ile Gly Leu Gly Glu Asp Gly Pro Thr His Gln Pro
   490                 495                 500                 505
   gtt gaa acc ttg gct gca ctg cgc gcc atc cca ggt ctg tcc gtc ctg    1767
   Val Glu Thr Leu Ala Ala Leu Arg Ala Ile Pro Gly Leu Ser Val Leu
                   510                 515                 520
   cgt cct gca gat gcg aac gag acc gcc cag gct tgg gct gca gca ctt    1815
   Arg Pro Ala Asp Ala Asn Glu Thr Ala Gln Ala Trp Ala Ala Ala Leu
               525                 530                 535
   gag tac aag gaa ggc cct aag ggt ctt gca ctg acc cgc cag aac gtt    1863
   Glu Tyr Lys Glu Gly Pro Lys Gly Leu Ala Leu Thr Arg Gln Asn Val
           540                 545                 550
   cct gtt ctg gaa ggc acc aag gag aag gct gct gaa ggc gtt cgc cgc    1911
   Pro Val Leu Glu Gly Thr Lys Glu Lys Ala Ala Glu Gly Val Arg Arg
       555                 560                 565
   ggt ggc tac gtc ctg gtt gag ggt tcc aag gaa acc cca gat gtg atc    1959
   Gly Gly Tyr Val Leu Val Glu Gly Ser Lys Glu Thr Pro Asp Val Ile
   570                 575                 580                 585
   ctc atg ggc tcc ggc tcc gag gtt cag ctt gca gtt aac gct gcg aag    2007
   Leu Met Gly Ser Gly Ser Glu Val Gln Leu Ala Val Asn Ala Ala Lys
                   590                 595                 600
   gct ctg gaa gct gag ggc gtt gca gct cgc gtt gtt tcc gtt cct tgc    2055
   Ala Leu Glu Ala Glu Gly Val Ala Ala Arg Val Val Ser Val Pro Cys
               605                 610                 615
   atg gat tgg ttc cag gag cag gac gca gag tac atc gag tcc gtt ctg    2103
   Met Asp Trp Phe Gln Glu Gln Asp Ala Glu Tyr Ile Glu Ser Val Leu
           620                 625                 630
   cct gca gct gtg acc gct cgt gtg tct gtt gaa gct ggc atc gca atg     2151
   Pro Ala Ala Val Thr Ala Arg Val Ser Val Glu Ala Gly Ile Ala Met
       635                 640                 645
   cct tgg tac cgc ttc ttg ggc acc cag ggc cgt gct gtc tcc ctt gag     2199
   Pro Trp Tyr Arg Phe Leu Gly Thr Gln Gly Arg Ala Val Ser Leu Glu
   650                 655                 660                 665
   cac ttc ggt gct tct gcg gat tac cag acc ctg ttt gag aag ttc ggc     2247
   His Phe Gly Ala Ser Ala Asp Tyr Gln Thr Leu Phe Glu Lys Phe Gly
                   670                 675                 680
   atc acc acc gat gca gtc gtg gca gcg gcc aag gac tcc att aac ggt     2295
   Ile Thr Thr Asp Ala Val Val Ala Ala Ala Lys Asp Ser Ile Asn Gly
               685                 690                 695
   taattgccct gctgttttta gcttcaaccc ggggcaatat gattctccgg aattt        2350
<210> 2
   <211> 697
   <212> PRT
   <213> Corynebacterium glutamicum
<400> 2
   Leu Thr Leu Ser Pro Glu Leu Gln Ala Leu Thr Val Arg Asn Tyr Pro
    1               5                  10                  15
   Ser Asp Trp Ser Asp Val Asp Thr Lys Ala Val Asp Thr Val Arg Val
               20                  25                  30
   Leu Ala Ala Asp Ala Val Glu Asn Cys Gly Ser Gly His Pro Gly Thr
           35                  40                  45
   Ala Met Ser Leu Ala Pro Leu Ala Tyr Thr Leu Tyr Gln Arg Val Met
       50                  55                  60
   Asn Val Asp Pro Gln Asp Thr Asn Trp Ala Gly Arg Asp Arg Phe Val
   65                  70                  75                  80
   Leu Ser Cys Gly His Ser Ser Leu Thr Gln Tyr Ile Gln Leu Tyr Leu
                   85                  90                  95
   Gly Gly Phe Gly Leu Glu Met Asp Asp Leu Lys Ala Leu Arg Thr Trp
               100                 105                 110
   Asp Ser Leu Thr Pro Gly His Pro Glu Tyr Arg His Thr Lys Gly Val
           115                 120                 125
   Glu Ile Thr Thr Gly Pro Leu Gly Gln Gly Leu Ala Ser Ala Val Gly
       130                 135                 140
   Met Ala Met Ala Ala Arg Arg Glu Arg Gly Leu Phe Asp Pro Thr Ala
   145                 150                 155                 160
   Ala Glu Gly Glu Ser Pro Phe Asp His His Ile Tyr Val Ile Ala Ser
                   165                 170                 175
   Asp Gly Asp Leu Gln Glu Gly Val Thr Ser Glu Ala Ser Ser Ile Ala
               180                 185                 190
   Gly Thr Gln Gln Leu Gly Asn Leu Ile Val Phe Trp Asp Asp Asn Arg
   195 200 205
   Ile Ser Ile Glu Asp Asn Thr Glu Ile Ala Phe Asn Glu Asp Val Val
   210 215 220
   Ala Arg Tyr Lys Ala Tyr Gly Trp Gln Thr Ile Glu Val Glu Ala Gly
   225 230 235 240
   Glu Asp Val Ala Ala Ile Glu Ala Ala Val Ala Glu Ala Lys Lys Asp
   245 250 255
   Thr Lys Arg Pro Thr Phe Ile Arg Val Arg Thr Ile Ile Gly Phe Pro
   260 265 270
   Ala Pro Thr Met Met Asn Thr Gly Ala Val His Gly Ala Ala Leu Gly
   275 280 285
   Ala Arg Glu Val Ala Ala Thr Lys Thr Glu Leu Gly Phe Asp Pro Glu
   290 295 300
   Ala His Phe Ala Ile Asp Asp Glu Val Ile Ala His Thr Arg Ser Leu
   305 310 315 320
   Ala Glu Arg Ala Ala Gln Lys Lys Ala Ala Trp Gln Val Lys Phe Asp
   325 330 335
   Glu Trp Ala Ala Ala Asn Pro Glu Asn Lys Ala Leu Phe Asp Arg Leu
   340 345 350
   Asn Ser Arg Glu Leu Pro Ala Gly Tyr Ala Asp Glu Leu Pro Thr Trp
   355 360 365
   Asp Ala Asp Glu Lys Gly Val Ala Thr Arg Lys Ala Ser Glu Ala Ala
   370 375 380
   Leu Gln Ala Leu Gly Lys Thr Leu Pro Glu Leu Trp Gly Gly Ser Ala
   385 390 395 400
   Asp Leu Ala Gly Ser Asn Asn Thr Val Ile Lys Gly Ser Pro Ser Phe
   405 410 415
   Gly Pro Glu Ser Ile Ser Thr Glu Thr Trp Ser Ala Glu Pro Tyr Gly
   420 425 430
   Arg Asn Leu His Phe Gly Ile Arg Glu His Ala Met Gly Ser Ile Leu
   435 440 445
   Asn Gly Ile Ser Leu His Gly Gly Thr Arg Pro Tyr Gly Gly Thr Phe
   450 455 460
   Leu Ile Phe Ser Asp Tyr Met Arg Pro Ala Val Arg Leu Ala Ala Leu
   465 470 475 480
   Met Glu Thr Asp Ala Tyr Tyr Val Trp Thr His Asp Ser Ile Gly Leu
   485 490 495
   Gly Glu Asp Gly Pro Thr His Gln Pro Val Glu Thr Leu Ala Ala Leu
   500 505 510
   Arg Ala Ile Pro Gly Leu Ser Val Leu Arg Pro Ala Asp Ala Asn Glu
   515 520 525
   Thr Ala Gln Ala Trp Ala Ala Ala Leu Glu Tyr Lys Glu Gly Pro Lys
   530 535 540
   Gly Leu Ala Leu Thr Arg Gln Asn Val Pro Val Leu Glu Gly Thr Lys
   545                 550                 555                 560
   Glu Lys Ala Ala Glu Gly Val Arg Arg Gly Gly Tyr Val Leu Val Glu
                   565                 570                 575
   Gly Ser Lys Glu Thr Pro Asp Val Ile Leu Met Gly Ser Gly Ser Glu
               580                 585                 590
   Val Gln Leu Ala Val Asn Ala Ala Lys Ala Leu Glu Ala Glu Gly Val
           595                 600                 605
   Ala Ala Arg Val Val Ser Val Pro Cys Met Asp Trp Phe Gln Glu Gln
       610                 615                 620
   Asp Ala Glu Tyr Ile Glu Ser Val Leu Pro Ala Ala Val Thr Ala Arg
   625                 630                 635                 640
   Val Ser Val Glu Ala Gly Ile Ala Met Pro Trp Tyr Arg Phe Leu Gly
                   645                 650                 655
   Thr Gln Gly Arg Ala Val Ser Leu Glu His Phe Gly Ala Ser Ala Asp
               660                 665                 670
   Tyr Gln Thr Leu Phe Glu Lys Phe Gly Ile Thr Thr Asp Ala Val Val
           675                 680                 685
   Ala Ala Ala Lys Asp Ser Ile Asn Gly
       690                 695
<210> 3
   <211> 2160
   <212> DNA
   <213> Corynebacterium glutamicum
<220>
   <221> CDS
   <222> (327)..(2063)
<400> 3
   ttagaggcga ttctgtgagg tcactttttg tggggtcggg gtctaaattt ggccagtttt 60
   cgaggcgacc agacaggcgt gcccacgatg tttaaatagg cgatcggtgg gcatctgtgt 120
   ttggtttcga cgggctgaaa ccaaaccaga ctgcccagca acgacggaaa tcccaaaagt 180
   gggcatccct gtttggtacc gagtacccac ccgggcctga aactccctgg caggcgggcg 240
   aagcgtggca acaactggaa tttaagagca caattgaagt cgcaccaagt taggcaacac 300
   aatagccata acgttgagga gttcag atg gca cac agc tac gca gaa caa tta  353
                                Met Ala His Ser Tyr Ala Glu Gln Leu
                                1               5
   att gac act ttg gaa gct caa ggt gtg aag cga att tat ggt ttg gtg   401
   Ile Asp Thr Leu Glu Ala Gln Gly Val Lys Arg Ile Tyr Gly Leu Val
   10                  15                  20                  25
   ggt gac agc ctt aat ccg atc gtg gat gct gtc cgc caa tca gat att   449
   Gly Asp Ser Leu Asn Pro Ile Val Asp Ala Val Arg Gln Ser Asp Ile
                   30                  35                  40
   gag tgg gtg cac gtt cga aat gag gaa gcg gcg gcg ttt gca gcc ggt     497
   Glu Trp Val His Val Arg Asn Glu Glu Ala Ala Ala Phe Ala Ala Gly
               45                  50                  55
   gcg gaa tcg ttg atc act ggg gag ctg gca gta tgt gct gct tct tgt     545
   Ala Glu Ser Leu Ile Thr Gly Glu Leu Ala Val Cys Ala Ala Ser Cys
           60                  65                  70
   ggt cct gga aac aca cac ctg att cag ggt ctt tat gat tcg cat cga     593
   Gly Pro Gly Asn Thr His Leu Ile Gln Gly Leu Tyr Asp Ser His Arg
       75                  80                  85
   aat ggt gcg aag gtg ttg gcc atc gct agc cat att ccg agt gcc cag     641
   Asn Gly Ala Lys Val Leu Ala Ile Ala Ser His Ile Pro Ser Ala Gln
   90                  95                  100                 105
   att ggt tcg acg ttc ttc cag gaa acg cat ccg gag att ttg ttt aag     689
   Ile Gly Ser Thr Phe Phe Gln Glu Thr His Pro Glu Ile Leu Phe Lys
                   110                 115                 120
   gaa tgc tct ggt tac tgc gag atg gtg aat ggt ggt gag cag ggt gaa     737
   Glu Cys Ser Gly Tyr Cys Glu Met Val Asn Gly Gly Glu Gln Gly Glu
               125                 130                 135
   cgc att ttg cat cac gcg att cag tcc acc atg gcg ggt aaa ggt gtg     785
   Arg Ile Leu His His Ala Ile Gln Ser Thr Met Ala Gly Lys Gly Val
           140                 145                 150
   tcg gtg gta gtg att cct ggt gat atc gct aag gaa gac gca ggt gac     833
   Ser Val Val Val Ile Pro Gly Asp Ile Ala Lys Glu Asp Ala Gly Asp
       155                 160                 165
   ggt act tat tcc aat tcc act att tct tct ggc act cct gtg gtg ttc     881
   Gly Thr Tyr Ser Asn Ser Thr Ile Ser Ser Gly Thr Pro Val Val Phe
   170                 175                 180                 185
   ccg gat cct act gag gct gca gcg ctg gtg gag gcg att aac aac gct     929
   Pro Asp Pro Thr Glu Ala Ala Ala Leu Val Glu Ala Ile Asn Asn Ala
                   190                 195                 200
   aag tct gtc act ttg ttc tgc ggt gcg ggc gtg aag aat gct cgc gcg     977
   Lys Ser Val Thr Leu Phe Cys Gly Ala Gly Val Lys Asn Ala Arg Ala
               205                 210                 215
   cag gtg ttg gag ttg gcg gag aag att aaa tca ccg atc ggg cat gcg     1025
   Gln Val Leu Glu Leu Ala Glu Lys Ile Lys Ser Pro Ile Gly His Ala
           220                 225                 230
   ctg ggt ggt aag cag tac atc cag cat gag aat ccg ttt gag gtc ggc     1073
   Leu Gly Gly Lys Gln Tyr Ile Gln His Glu Asn Pro Phe Glu Val Gly
       235                 240                 245
   atg tct ggc ctg ctt ggt tac ggc gcc tgc gtg gat gcg tcc aat gag     1121
   Met Ser Gly Leu Leu Gly Tyr Gly Ala Cys Val Asp Ala Ser Asn Glu
   250                 255                 260                 265
   gcg gat ctg ctg att cta ttg ggt acg gat ttc cct tat tct gat ttc     1169
   Ala Asp Leu Leu Ile Leu Leu Gly Thr Asp Phe Pro Tyr Ser Asp Phe
                   270                 275                 280
   ctt cct aaa gac aac gtt gcc cag gtg gat atc aac ggt gcg cac att     1217
   Leu Pro Lys Asp Asn Val Ala Gln Val Asp Ile Asn Gly Ala His Ile
               285                 290                 295
   ggt cga cgt acc acg gtg aag tat ccg gtg acc ggt gat gtt gct gca     1265
   Gly Arg Arg Thr Thr Val Lys Tyr Pro Val Thr Gly Asp Val Ala Ala
           300                 305                 310
   aca atc gaa aat att ttg cct cat gtg aag gaa aaa aca gat cgt tcc     1313
   Thr Ile Glu Asn Ile Leu Pro His Val Lys Glu Lys Thr Asp Arg Ser
       315                 320                 325
   ttc ctt gat cgg atg ctc aag gca cac gag cgt aag ttg agc tcg gtg     1361
   Phe Leu Asp Arg Met Leu Lys Ala His Glu Arg Lys Leu Ser Ser Val
   330                 335                 340                 345
   gta gag acg tac aca cat aac gtc gag aag cat gtg cct att cac cct     1409
   Val Glu Thr Tyr Thr His Asn Val Glu Lys His Val Pro Ile His Pro
                   350                 355                 360
   gaa tac gtt gcc tct att ttg aac gag ctg gcg gat aag gat gcg gtg     1457
   Glu Tyr Val Ala Ser Ile Leu Asn Glu Leu Ala Asp Lys Asp Ala Val
               365                 370                 375
   ttt act gtg gat acc ggc atg tgc aat gtg tgg cat gcg agg tac atc     1505
   Phe Thr Val Asp Thr Gly Met Cys Asn Val Trp His Ala Arg Tyr Ile
           380                 385                 390
   gag aat ccg gag gga acg cgc gac ttt gtg ggt tca ttc cgc cac ggc     1553
   Glu Asn Pro Glu Gly Thr Arg Asp Phe Val Gly Ser Phe Arg His Gly
       395                 400                 405
   acg atg gct aat gcg ttg cct cat gcg att ggt gcg caa agt gtt gat     1601
   Thr Met Ala Asn Ala Leu Pro His Ala Ile Gly Ala Gln Ser Val Asp
   410                 415                 420                 425
   cga aac cgc cag gtg atc gcg atg tgt ggc gat ggt ggt ttg ggc atg     1649
   Arg Asn Arg Gln Val Ile Ala Met Cys Gly Asp Gly Gly Leu Gly Met
                   430                 435                 440
   ctg ctg ggt gag ctt ctg acc gtt aag ctg cac caa ctt ccg ctg aag     1697
   Leu Leu Gly Glu Leu Leu Thr Val Lys Leu His Gln Leu Pro Leu Lys
               445                 450                 455
   gct gtg gtg ttt aac aac agt tct ttg ggc atg gtg aag ttg gag atg     1745
   Ala Val Val Phe Asn Asn Ser Ser Leu Gly Met Val Lys Leu Glu Met
           460                 465                 470
   ctc gtg gag gga cag cca gaa ttt ggt act gac cat gag gaa gtg aat     1793
   Leu Val Glu Gly Gln Pro Glu Phe Gly Thr Asp His Glu Glu Val Asn
       475                 480                 485
   ttc gca gag att gcg gcg gct gcg ggt atc aaa tcg gta cgc atc acc     1841
   Phe Ala Glu Ile Ala Ala Ala Ala Gly Ile Lys Ser Val Arg Ile Thr
   490                 495                 500                 505
   gat ccg aag aaa gtt cgc gag cag cta gct gag gca ttg gca tat cct     1889
   Asp Pro Lys Lys Val Arg Glu Gln Leu Ala Glu Ala Leu Ala Tyr Pro
                   510                 515                 520
   gga cct gta ctg atc gat atc gtc acg gat cct aat gcg ctg tcg atc     1937
   Gly Pro Val Leu Ile Asp Ile Val Thr Asp Pro Asn Ala Leu Ser Ile
               525                 530                 535
   cca cca acc atc acg tgg gaa cag gtc atg gga ttc agc aag gcg gcc     1985
   Pro Pro Thr Ile Thr Trp Glu Gln Val Met Gly Phe Ser Lys Ala Ala
           540                 545                 550
   acc cga acc gtc ttt ggt gga gga gta gga gcg atg atc gat ctg gcc   2033
   Thr Arg Thr Val Phe Gly Gly Gly Val Gly Ala Met Ile Asp Leu Ala
       555                 560                 565
   cgt tcg aac ata agg aat att cct act cca tgatgattga tacacctgct     2083
   Arg Ser Asn Ile Arg Asn Ile Pro Thr Pro
   570                 575
   gttctcattg accgcgagcg cttaactgcc aacatttcca ggatggcagc tcacgccggt 2143
   gcccatgaga ttgccct                                                2160
<210> 4
   <211> 579
   <212> PRT
   <213> Corynebacterium glutamicum
<400> 4
   Met Ala His Ser Tyr Ala Glu Gln Leu Ile Asp Thr Leu Glu Ala Gln
    1               5                  10                  15
   Gly Val Lys Arg Ile Tyr Gly Leu Val Gly Asp Ser Leu Asn Pro Ile
               20                  25                  30
   Val Asp Ala Val Arg Gln Ser Asp Ile Glu Trp Val His Val Arg Asn
           35                  40                  45
   Glu Glu Ala Ala Ala Phe Ala Ala Gly Ala Glu Ser Leu Ile Thr Gly
       50                  55                  60
   Glu Leu Ala Val Cys Ala Ala Ser Cys Gly Pro Gly Asn Thr His Leu
   65                  70                  75                  80
   Ile Gln Gly Leu Tyr Asp Ser His Arg Asn Gly Ala Lys Val Leu Ala
                   85                  90                  95
   Ile Ala Ser His Ile Pro Ser Ala Gln Ile Gly Ser Thr Phe Phe Gln
               100                 105                 110
   Glu Thr His Pro Glu Ile Leu Phe Lys Glu Cys Ser Gly Tyr Cys Glu
           115                 120                 125
   Met Val Asn Gly Gly Glu Gln Gly Glu Arg Ile Leu His His Ala Ile
       130                 135                 140
   Gln Ser Thr Met Ala Gly Lys Gly Val Ser Val Val Val Ile Pro Gly
   145                 150                 155                 160
   Asp Ile Ala Lys Glu Asp Ala Gly Asp Gly Thr Tyr Ser Asn Ser Thr
                   165                 170                 175
   Ile Ser Ser Gly Thr Pro Val Val Phe Pro Asp Pro Thr Glu Ala Ala
               180                 185                 190
   Ala Leu Val Glu Ala Ile Asn Asn Ala Lys Ser Val Thr Leu Phe Cys
           195                 200                 205
   Gly Ala Gly Val Lys Asn Ala Arg Ala Gln Val Leu Glu Leu Ala Glu
       210                 215                 220
   Lys Ile Lys Ser Pro Ile Gly His Ala Leu Gly Gly Lys Gln Tyr Ile
   225                 230                 235                 240
   Gln His Glu Asn Pro Phe Glu Val Gly Met Ser Gly Leu Leu Gly Tyr
                   245                 250                 255
   Gly Ala Cys Val Asp Ala Ser Asn Glu Ala Asp Leu Leu Ile Leu Leu
               260                 265                 270
   Gly Thr Asp Phe Pro Tyr Ser Asp Phe Leu Pro Lys Asp Asn Val Ala
           275                 280                 285
   Gln Val Asp Ile Asn Gly Ala His Ile Gly Arg Arg Thr Thr Val Lys
       290                 295                 300
   Tyr Pro Val Thr Gly Asp Val Ala Ala Thr Ile Glu Asn Ile Leu Pro
   305                 310                 315                 320
   His Val Lys Glu Lys Thr Asp Arg Ser Phe Leu Asp Arg Met Leu Lys
                   325                 330                 335
   Ala His Glu Arg Lys Leu Ser Ser Val Val Glu Thr Tyr Thr His Asn
               340                 345                 350
   Val Glu Lys His Val Pro Ile His Pro Glu Tyr Val Ala Ser Ile Leu
           355                 360                 365
   Asn Glu Leu Ala Asp Lys Asp Ala Val Phe Thr Val Asp Thr Gly Met
       370                 375                 380
   Cys Asn Val Trp His Ala Arg Tyr Ile Glu Asn Pro Glu Gly Thr Arg
   385                 390                 395                 400
   Asp Phe Val Gly Ser Phe Arg His Gly Thr Met Ala Asn Ala Leu Pro
                   405                 410                 415
   His Ala Ile Gly Ala Gln Ser Val Asp Arg Asn Arg Gln Val Ile Ala
               420                 425                 430
   Met Cys Gly Asp Gly Gly Leu Gly Met Leu Leu Gly Glu Leu Leu Thr
           435                 440                 445
   Val Lys Leu His Gln Leu Pro Leu Lys Ala Val Val Phe Asn Asn Ser
       450                 455                 460
   Ser Leu Gly Met Val Lys Leu Glu Met Leu Val Glu Gly Gln Pro Glu
   465                 470                 475                 480
   Phe Gly Thr Asp His Glu Glu Val Asn Phe Ala Glu Ile Ala Ala Ala
                   485                 490                 495
   Ala Gly Ile Lys Ser Val Arg Ile Thr Asp Pro Lys Lys Val Arg Glu
               500                 505                 510
   Gln Leu Ala Glu Ala Leu Ala Tyr Pro Gly Pro Val Leu Ile Asp Ile
           515                 520                 525
   Val Thr Asp Pro Asn Ala Leu Ser Ile Pro Pro Thr Ile Thr Trp Glu
       530                 535                 540
   Gln Val Met Gly Phe Ser Lys Ala Ala Thr Arg Thr Val Phe Gly Gly
   545                 550                 555                 560
   Gly Val Gly Ala Met Ile Asp Leu Ala Arg Ser Asn Ile Arg Asn Ile
                   565                 570                 575
   Pro Thr Pro
<210> 5
   <211> 875
   <212> DNA
   <213> Corynebacterium glutamicum
<400> 5
   tgcgagatgg tgaatggtgg tgagcagggt gaacgcattt tgcatcacgc gattcagtcc 60
   accatggcgg gtaaaggtgt gtcggtggta gtgattcctg gtgatatcgc taaggaagac 120
   gcaggtgacg gtacttattc caattccact atttcttctg gcactcctgt ggtgttcccg 180
   gatcctactg aggctgcagc gctggtggag gcgattaaca acgctaagtc tgtcactttg 240
   ttctgcggtg cgggcgtgaa gaatgctcgc gcgcaggtgt tggagttggc ggagaagatt 300
   aaatcaccga tcgggcatgc gctgggtggt aagcagtaca tccagcatga gaatccgttt 360
   gaggtcggca tgtctggcct gcttggttac ggcgcctgcg tggatgcgtc caatgaggcg 420
   gatctgctga ttctattggg tacggatttc ccttattctg atttccttcc taaagacaac 480
   gttgcccagg tggatatcaa cggtgcgcac attggtcgac gtaccacggt gaagtatccg 540
   gtgaccggtg atgttgctgc aacaatcgaa aatattttgc ctcatgtgaa ggaaaaaaca 600
   gatcgttcct tccttgatcg gatgctcaag gcacacgagc gtaagttgag ctcggtggta 660
   gagacgtaca cacataacgt cgagaagcat gtgcctattc accctgaata cgttgcctct 720
   attttgaacg agctggcgga taaggatgcg gtgtttactg tggataccgg catgtgcaat 780
   gtgtggcatg cgaggtacat cgagaatccg gagggaacgc gcgactttgt gggttcattc 840
   cgccacggca cgatggctaa tgcgttgcct catgc                           875